# EUROPÄISCHE PATENTSCHRIFT

(11) **EP 2 092 907 B1**
(45) Veröffentlichungstag und Bekanntmachung des Hinweises auf die Patenterteilung: **16.02.2011**
(21) Anmeldenummer: 08151747.6
(22) Anmeldetag: 21.02.2008
(51) Int. Cl.: A61B 19/00

(54) **Lageberechnung von Körperteilen unter Berücksichtigung der anatomischen Symmetrie**
Calculation of the position of body parts considering anatomical symmetry
Calcul de la position de parties du corps en fonction de la symétrie anatomique

(43) Veröffentlichungstag der Anmeldung: 26.08.2009
(73) Patentinhaber: BrainLAB AG, 85622 Feldkirchen (DE)
(72) Erfinder: Warkentine, Blaine, 84020 Draper, UT (US); Huber, Hansjoerg, 80805 München (DE)
(74) Vertreter: Schwabe - Sandmair - Marx

(56) Entgegenhaltungen:
- WO-A-01/35842
- DE-A1-102006 056 399
- FR-A- 2 880 791
- US-A1- 2004 106 861
- DATABASE MEDLINE [Online] US NATIONAL LIBRARY OF MEDICINE (NLM), BETHESDA, MD, US; September 1995 (1995-09), ADAMS D J ET AL: "Three-dimensional geometric and structural symmetry of the turkey ulna." XP002488556 Database accession no. NLM7472747 & JOURNAL OF ORTHOPAEDIC RESEARCH : OFFICIAL PUBLICATION OF THE ORTHOPAEDIC RESEARCH SOCIETY SEP 1995, Bd. 13, Nr. 5, September 1995 (1995-09), Seiten 690-699, ISSN: 0736-0266

## Beschreibung

Die vorliegende Erfindung betrifft die Bestimmung der Lage von Körperteilen, die mit einem Gelenk eines anatomischen Körpers, z.B. eines Menschen oder eines Tieres verbunden sind. Bei dem Gelenk kann es sich beispielsweise um ein Kniegelenk, Schultergelenk, Hüftgelenk usw. handeln. Diese Gelenke haben die Eigenschaft, dass es jeweils ein linkes und rechtes Gelenk gibt, die eine symmetrische Kinematik aufweisen, d.h. die möglichen Bewegungsabläufe sind im Idealfall eines gesunden Körpers symmetrisch, insbesondere bzgl. einer Symmetrieebene und/oder einer Symmetrieachse. Die Erfindung nutzt die kinematische Symmetrie der Gelenke, um aus der Lage von Körperteilen, die mit einem Gelenk auf einer Seite des Körpers verbunden sind, auf eine mögliche oder gewünschte Lage von Körperteilen, die mit dem dazu symmetrischen Gelenk auf der anderen Seite des Körpers verbunden sind, zu schließen. Zur Kinematik der Gelenke sei auf das Standardwerk von I. A. Kapandji "Funktionelle Anatomie der Gelenke" (englischer Titel: "The Physiology of the Joints") hingewiesen, in dem die menschliche Biomechanik einschließlich der Kinematik der Gelenke beschrieben wird.

Die vorliegende Erfindung betrifft weiter nicht nur die Nutzung der anatomischen Symmetrie soweit sie die Kinematik der Bewegung betrifft sondern auch oder alternativ die Nutzung der anatomischen Symmetrie soweit sie die statische Lage der Körperteile betrifft. Insbesondere wird die statische Symmetrie genutzt, um die Lage von Körperteilen oder die relative Lage von Körperteilen zu bestimmen. Im Idealfall ist der anatomische Körper symmetrisch zu einer Symmetrieebene, der medianen Sagittalebene. Basierend auf dieser ideellen Annahme, kann die Lage eines Körperteils berechnet werden, die es bei idealer Symmetrie einnehmen würde. Auch kann berechnet werden, wie die relative Lage der Körperteile bei idealer anatomischer (statischer) Symmetrie wäre.

Ein Verfahren zur Bestimmung des Femurverankerungspunktes des Kreuzbandes eines Kniegelenkes ist in der europäischen Patentschrift DE 693 19 212 T2 beschrieben. Die Vermessung eines Antetorsionswinkels eines Femurs ist aus US 2007/0161929 A1 sowie aus EP 1788 581 A1 bekannt. Bezüglich der Bestimmung von Achsen und Orientierungen eines Körperteils und der Zuordnung von Bezugssystemen zu einem Körperteil wird auch auf diese Druckschriften verwiesen.

WO 01/35842 A offenbart ein Verfahren und eine Vorrichtung zur Angleichung der Lage eines gebrochenen Knochens an die bezüglich der medialen Sagittalebene symmetrisch gelegene Lage eines gesunden entsprechenden Knochens. Diese Schrift offenbart keine Veränderung der Relativstellung weder des gesunden noch des verletzten Körperteils zur Überprüfung der Kinematik bzw. funktioneller Bewegungsabläufe im verletzten Körperteil verglichen mit dem gesunden Körperteil.

FR-A-2 880 791 offenbart ein Verfahren und ein System zur computerunterstützten Richtigstellung einer Fraktur, wobei die Knochenfragmente des Patienten in erste und zweite Segmente aufgeteilt werden, die symmetrisch zu einem dritten Knochensegment. Aus den Eigenschaften des dritten Knochenfragments lässt sich gemäß dieser Schrift eine Endposition der Fragmente des verletzten Knochens ermitteln, die diese nach erfolgter Reposition einnehmen sollen. Diese Schrift gibt jedoch keinerlei Hinweis darauf, dass für ein Körperteil, der alle Bestandteil eines Gelenks umfasst, in seiner Beweglichkeit mit einem Verfahren gemäß der vorliegenden Erfindung untersucht werden könnte.

DATABASE MEDLINE [Online] US NATIONAL LIBRARY OF MEDICINE (NLM), BETHESDA, MD, US; ADAMS D J ET AL: "Three-dimensional geometric and structural symmetry of the turkey ulna" befasst sich mit der Ausnutzung von Symmetrien für paarweise angeordnete lange Röhrenknochen, bezieht sich allerdings im Übrigen auf die Vergleiche physikalischer Parameter der Knochen wie z.B. der Hauptträgheitsmomente und der Krümmung der Knochen.

DE 10 2006 056399 A1 ist auf ein Funktionsgelenk-Arthroplastikverfahren gerichtet, das sich hauptsächlich mit dem Einbringen eines Implantats, beispielsweise einer Kreuzbandplastik in ein Kniegelenk. Dazu offenbart diese Schrift eine Distraktionsvorrichtung, die das Präparieren des Gelenks zur Aufnahme des gewählten Implantats erleichtert. Ferner beschreibt diese Schrift die Möglichkeit, die Kinematik eines verletzten Gelenks bezüglich des unverletzten Zustands wiederherzustellen, indem biomechanische Daten des geschlossenen verletzten Gelenks bestimmt und aufgezeichnet werden oder in dem die normale Kinematik des verletzten Gelenks mit der Kinematik des unverletzten Gelenks verglichen wird. Ein Vergleich mehrerer statischer Positionen bzw. Relativstellungen des verletzten und unverletzten Körperteils wird nicht nahegelegt.

Aufgabe der Erfindung ist es die Lage oder Relativlage von Körperteilen unter Berücksichtigung der im Idealfall gegebenen anatomischen Symmetrie eines anatomischen Körpers zu bestimmen.

Aufgabe der Erfindung ist es insbesondere die Lage zu bestimmen, die ein Körperteil einnimmt oder einnehmen würde, wenn das damit verbundene Gelenk eine Kinematik hätte, die symmetrisch zu dem entsprechenden Gelenk, das auf der anderen Seite des Körpers angeordnet ist, ist.

Die vorstehende Aufgabe wird durch den Gegenstand des unabhängigen Anspruchs gelöst. Vorteilhafte Weiterbildungen gehen aus den Unteransprüchen hervor.

Die Erfindung soll insbesondere unterstützen bei dem Erkennen und Quantifizieren von Fehlstellungen von Körperteilen, die sich aus anatomischen Symmetriebetrachtungen ergeben. Hierbei ist es natürlich dem Arzt überlassen, zu beurteilen, ob es sich um eine Fehlstellung handelt und welche der beiden Seiten des Körpers mit einer Fehlstellung behaftet sind und welche Seite eine gesunde Stellung darstellt. Auch kann die Erfindung helfen bei der Beurteilung von Relativstellungen von Körperteilen. Beispielsweise kann das Ausmaß eines Varus oder Valgus auf einer Seite mit dem Ausmaß eines Varus oder Valgus auf der anderen Seiten eines anatomischen Körpers verglichen werden. Auch kann beispielsweise die maximale Abduktion des Femurs auf der einen Seite mit der maximalen Abduktion des Femurs auf der anderen Seite mittels der Erfindung verglichen werden. Insbesondere ist es möglich zu bestimmen, wo sich ein Körperteil nach einer Bewegung um ein Gelenk befinden würde, wenn es sich symmetrisch zu dem ihm anatomisch entsprechenden Körperteil verhalten würde.

Die Erfindung betrifft die Berechnung der Lage oder Relativlage von Körperteilen unter Anwendung von Symmetrieregeln. Die Symmetrieregeln basieren insbesondere auf der Bereitstellung von Daten (Symmetrieebenendaten), die die Lage einer Symmetrieebene des Körpers, die hierin auch als Körpersymmetrieebene bezeichnet wird, insbesondere relativ zu den Körperteilen und/oder in einem gemeinsamen Bezugssystem bestimmen oder implizieren. Die Lage einer Symmetrieebene wird insbesondere impliziert, wenn vorausgesetzt wird, dass zwei sich anatomisch entsprechende Körperteile (z.B. ein linker und rechter Oberschenkel), deren Lage durch Körperteildaten (siehe unten) beschrieben wird, symmetrisch zu der Symmetrieebene angeordnet sind. Ein erstseitiges Körperteil ist dabei ein Körperteil, das an einer der beiden durch die Körpersymmetrieebene aufgeteilten Seiten des anatomischen Körpers liegt. Das zweitseitige Körperteil liegt auf der anderen Seite. Erstseitige Körperteildaten beschreiben die Lage des mindestens einen erstseitigen Körperteils. Die Erfindung kann also auf ein, zwei, drei oder mehr Körperteile auf jeder Seite angewendet werden. Entsprechend beschreiben die zweitseitigen Körperteile-daten die Lage des mindestens einen zweitseitigen Körperteils. Die Lage eines Körperteils oder von zwei oder mehr Körperteilen wird vorzugsweise an der Körpersymmetrieebene gespiegelt. Vorzugsweise wird die gespiegelte Lage eines zweitseitigen Körperteils mit der Lage des erstseitigen Körperteils verglichen, das anatomisch dem zweitseitigen Körperteil entspricht. Insbesondere wird eine Abweichung der gespiegelten Lage des zweitseitigen Körperteils von der Lage des anatomisch entsprechenden erstseitigen Körperteils bestimmt und/oder angezeigt. Die Anzeige erfolgt insbesondere überlappend, so dass für den Betrachter sowohl die gespiegelte Lage des zweitseitigen Körperteils als auch die ungespiegelte Lage des erstseitigen Körperteils sichtbar sind. Die Anzeige kann gleichzeitig erfolgen, oder der Betrachter kann zwischen den beiden Abbildungen hin und herschalten, oder sie werden beispielsweise nebeneinander angeordnet.

Zusätzlich oder alternativ zu einem Vergleich einer ungespiegelten Lage mit einer gespiegelten Lage werden die Lagen zumindest zweier zweitseitiger Körperteile miteinander verglichen, insbesondere wird hierzu eine Relativlage der mindestens zwei zweitseitigen Körperteile bestimmt oder bereitgestellt. Die Bestimmung der gespiegelten Relativlage basiert auf den zweitseitigen Körperteildaten und den Symmetrieebenendaten.

Die vorgenannte Bestimmung der gespiegelten Relativlage kann auf verschiedene Weisen erfolgen. Beispielsweise kann die Lage eines ersten zweitseitigen Körperteils gespiegelt werden und die Lage eines zweiten zweitseitigen Körperteils kann ebenfalls gespiegelt werden. Dann wird die relative Lage zwischen der gespiegelten Lage des ersten zweitseitigen Körperteils und der gespiegelten Lage des zweiten zweitseitigen Körperteils berechnet. Dies ist dann die gespiegelte Relativlage. Alternativ hierzu kann eine Relativgröße bestimmt werden, die die relative Lage des ersten zweitseitigen Körperteils relativ zum zweiten zweitseitigen Körperteils (auf der zweiten Seite der Körpersymmetrieebene) beschreibt. Diese Relativgröße kann beispielsweise ein Vektor sein oder es können auch zwei Vektoren sein. Im Falle von zwei Vektoren werden beispielsweise durch einen Vektor die Anfangspunkte einer die Lage der zweitseitigen Körperteile beschreibenden Strecke verknüpft und durch den zweiten Vektor werden die Endpunkte einer die Lage der zweitseitigen Körperteile beschreibenden Strecke verknüpft. Dieser Vektor oder diese Vektoren werden dann an der Symmetrieebene gespiegelt. Der gespiegelte Vektor oder die gespiegelten Vektoren stellen dann die gespiegelte Relativlage dar.

Die Relativlage kann nicht nur die relative Lage zwischen zwei verschiedenen Körperteilen beschreiben sondern auch die relative Lage, die ein und dasselbe Körperteil einnimmt, wenn es zwei verschiedene Stellungen (Lagen) hat. Beispielsweise kann eine Lage die Lage des Unterschenkels bei Extension des Beins darstellen und die andere Lage die Lage des Unterschenkels bei Flexion (z.B. 90°-Flexion). Das Körperteil (Unterschenkel) nimmt somit zwei verschiedene Stellungen ein. Die relative Lage zwischen diesen beiden verschiedenen Stellungen wird hierin auch als "Stellungs-Relativlage" bezeichnet. Vorzugsweise wird eine gespiegelte Stellungs-Relativlage gemäß einer Ausführungsform berechnet. Dies kann beispielsweise so erfolgen, dass die Lage des Körperteils in einer ersten Stellung gespiegelt wird und die Lage des Körperteils in einer zweiten Stellung ebenfalls gespiegelt wird. Die beiden verschiedenen gespiegelten Lagen, die das Körperteil in zwei verschiedenen gespiegelten Stellungen darstellen, haben eine relative Lage zueinander, die die gespiegelte Stellungs-Relativlage darstellt. Gemäß einer anderen Vorgehensweise kann die relative Lage zwischen zwei Stellungen des Körperteils durch eine Relativgröße beschrieben werden. Hierbei handelt es sich beispielsweise um einen oder um mehrere Vektoren, wie dies bereits oben erläutert wurde. Diese Relativgröße (Vektor oder Vektoren) wird dann an der Symmetrieebene gespiegelt. Die gespiegelte Relativgröße beschreibt dann die gespiegelte Stellungs-Relativlage. Die auf eine Seite gespiegelte Stellungs-Relativlage kann gemäß einer Ausführungsform dann beispielsweise mit der auf dieser Seite gegebenen Stellungs-Relativlage verglichen werden, um so Abweichungen von einer idealen anatomischen Symmetrie zu bestimmen. Gemäß einer besonderen Ausführungsform kann anhand der gespiegelten Stellungs-Relativlage insbesondere bestimmt werden, wie sich das anatomisch entsprechende Körperteil (beispielsweise der linke Oberschenkel) bei einem Übergang von einer ersten zur zweiten Stellung verhalten würde, wenn es die an der Körpersymmetrieebene gespiegelte Bewegung des anatomisch entsprechenden Körperteils auf der anderen Körperseite durchmachen würde. Beispielsweise werden hierzu ein oder zwei Vektoren an dem (erstseitigen) Körperteil angesetzt, die die gespiegelte Stellungs-Relativlage beschreiben. Beispielsweise wird ein Vektor an dem Anfang einer Strecke angesetzt, die die Lage des Körperteils darstellt, während der andere Vektor am Ende dieser Strecke angesetzt wird. Verbindet man dann die Spitze der Vektoren, so ergibt sich eine Strecke, die die Stellung des Körperteils darstellt, wenn es sich genauso wie das anatomisch entsprechende Körperteil bei einer Bewegung verhalten würde. Aufgrund der besonderen Bedeutung dieser Zielsetzung ist zusätzlich ein unabhängiger Anspruch darauf gerichtet. Die entsprechende Ausführungsform wird weiter unten erläutert.

Die so bestimmte Stellung des Körperteils kann dann wiederum mit der tatsächlichen Stellung des Körperteils verglichen werden. Somit können insbesondere Unterschiede in den Bewegungsabläufen oder in den Stellungs-Relativlagen zwischen der einen Seite und der anderen Seite des Körpers erkannt werden.

Bei einer der Ausführungsformen umfasst die Berechnung der gespiegelten Lage oder der gespiegelten Lagen und/oder der gespiegelten Relativlagen den Schritt, die Lage bzw. Relativlage in eine Ebene zu projizieren. Diese Ebene wird hierin als Projektionsebene bezeichnet. Interessiert man sich beispielsweise für den Varus oder Valgus eines Beins, so kann dieser durch eine Relativgröße beschrieben werden. Die Relativgröße ist insbesondere ein Winkel, der zwischen dem Unterschenkel und dem Oberschenkel ausgebildet ist, wenn sich das Bein in Extension befindet. Dieser Winkel kann dann beispielsweise aus den in die Projektionsebene projizierten Lagen bestimmt werden. Als Projektionsebene wird hier die so genannte Frontalebene oder koronale Ebene bevorzugt, die senkrecht zur Sagittalebene ist. Die projizierte Lage kann dann an der Körpersymmetrieebene gespiegelt werden. Es kann beispielsweise auch anders vorgegangen werden. So werden zuerst die unprojizierten Lagen gespiegelt werden und dann die gespiegelten Lagen in die Projektionsebene projiziert werden.

Danach wird dann insbesondere eine Relativgröße, wie beispielsweise der Winkel zwischen den beiden Körperteilen in der Projektionsebene bestimmt.

Die Lage eines Körperteils wird beispielsweise, wie schon erwähnt, durch eine Strecke dargestellt. Die Endpunkte der Strecke können beispielsweise über Landmarken bestimmt werden. Auch kann die Lage eines Körperteils durch charakteristische Achsen, wie z.B. eine Tibiaachse oder durch Ebenen, z.B. eine auf dem Acetabulum virtuell aufliegende Ebene beschrieben werden. Letztendlich können auch ein generisches Modell des Körperteils oder dreidimensionale Körperteildaten, die beispielsweise mittels Kernspin oder einer dreidimensionalen Röntgenaufnahme (CT) gewonnen werden, verwendet werden, um die Lage des Körperteils zu beschreiben.

Die Erfindung soll insbesondere Anwendung finden bei der Planung von Eingriffen in ein Körperteil, insbesondere Knochen, beispielsweise bei der Planung von Eingriffsorten, wie der Ort eines Verankerungspunktes oder der Ort einer Bohrung in dem Körperteil. Insbesondere soll sie bei der Planung von Eingriffes helfen, die einen Einfluss auf die spätere Kinematik des mit dem Körperteil verbundenen Gelenks haben, wie beispielsweise eine Kreuzbandoperation oder wie eine Implantation künstlicher Gelenke. Im letzten Fall sind die mit dem Gelenk verbundenen Körperteile zumindest zum Teil künstlich und nicht natürlich.

Vorteilhaft an der Erfindung ist, dass durch die Untersuchung der Kinematik eines gesunden Gelenkes, insbesondere basierend auf Relativstellungen, die Körperteile eines gesunden Gelenks einnehmen, es möglich wird, auf eine gewünschte Kinematik, insbesondere gewünschte Relativstellungen (Soll-Relativstellungen oder Soll-Neutralstellungen) von Körperteilen, die mit einem kranken Gelenk verbunden sind und deshalb eine oder mehrere gewünschte Relativstellungen (von sich aus) nicht oder nicht stabil einnehmen, zu schließen. Vorteilhaft kann die nichtideale Kinematik eines kranken Gelenks erkannt werden und/oder verhindert werden, dass diese zu einer Fehlplanung der Operation, insbesondere zu einer fehlerhaften Bestimmung des Eingriffsortes führt. Auch kann erkannt werden, wenn die Körperteile eine Relativstellung (z.B. so genannte "vordere Schublade" beim Kreuzbandriss) einnehmen, die der eines gesunden Gelenks nicht entspricht.

Vorteilhaft kann nach einer Operation durch die vorliegende Erfindung überprüft werden, ob die durch die Operation erzielte Kinematik des operierten Gelenks der gewünschten Kinematik entspricht. Die Kinematik lässt sich dabei vorteilhaft durch mindestens zwei, vorzugsweise drei oder mehr Relativstellungen beschreiben und erfassen. Das erfindungsgemäße Verfahren dient der Berechnung der Lage von Körperteilen, die mit kinematisch symmetrischen Gelenken verbunden sind. Beispielsweise ist die Flexions-Extensions-Bewegung des linken Kniegelenks symmetrisch zu der Flexions-Extensions-Bewegung des rechten Kniegelenks. Beispielsweise ist das Anheben des linken Oberarms nach links außen symmetrisch zum Anheben des rechten Oberarms nach rechts außen. Insbesondere kann die mediane Sagittalebene des anatomischen Körpers als Symmetrieebene (Körpersymmetrieebene) herangezogen werden, um aus der relativen Lage zweier mit einem Gelenk verbundenen Körperteile auf die dazu symmetrische Lage der Körperteile, die mit dem Gelenk auf der anderen Seite des Körpers verbunden sind, zu schließen. Kinematisch symmetrische Gelenke eines Körpers sind somit gegeben, falls die mit den zwei Gelenken (jeweils ein Gelenk auf jeder Körperseite) verbundenen Körperteile bei Betätigung der zwei Gelenke, um eine gleiche Bewegung (z.B. Flexion, Extension; Adduktion, Abduktion; Innenrotation, Außenrotation; Inklination, Reklination, etc.) zu erzielen, einen symmetrischen Bewegungsablauf aufweisen.

Im Folgenden wird die eine Seite des Körpers, also beispielsweise die Seite links von einer Symmetrielinie oder -ebene, z.B. der medianen Sagittalebene als erstseitig bezeichnet und die andere Seite des Körpers, also beispielsweise die Seite rechts von der Symmetrielinie oder - ebene, z.B. der medianen Sagittalebene als zweitseitig bezeichnet. Die erste Seite kann sich somit auf links oder rechts beziehen, und die zweite Seite auf die jeweils anderen Seite. Die Kinematik des Gelenks auf der ersten Seite ist symmetrisch zur Kinematik des entsprechenden Gelenks auf der zweiten Seite, im Falle eines ideal gesunden Körpers.

Vorteilhaft kann durch das erfindungsgemäße Verfahren die Lage eines Körperteils auf einer ersten Seite, also eines erstseitigen Körperteils (z.B. linker Unterschenkel) berechnet werden, indem insbesondere die Lage oder relative Lage der Köperteile auf der anderen Seite des Körpers, also der zweitseitigen Köperteile (z.B. rechten Ober- und Unterschenkel), die mit dem zweitseitigen Gelenk, z.B. rechten Kniegelenk, verbunden sind, berechnet werden.

Beispiele für ein erstes und zweites erstseitiges Körperteil sind der linke Ober- und Unterschenkel. Beispiele für ein erstes und zweites zweitseitiges Körperteil sind der rechte Ober- und Unterschenkel. Die erstseitigen Körperteile sind durch ein erstseitiges Gelenk, also z.B. das linke Kniegelenk verbunden. Zu diesem erstseitigen Gelenk gibt es ein symmetrisches zweitseitiges Gelenk, also z.B. das rechte Kniegelenk. Insbesondere gibt es eine anatomische Entsprechung (symmetrische anatomische Funktion) zwischen dem ersten erstseitigen Körperteil und dem ersten zweitseitigen Körperteil (z.B. bei beiden handelt es sich um einen Oberschenkel). Weiter gibt es insbesondere eine anatomische Entsprechung zwischen dem zweiten erstseitigen Körperteil und dem zweiten zweitseitigen Körperteil (z.B. handelt es sich bei beiden um Unterschenkel).

Für Rotationsbewegungen, beispielsweise Außenrotation und Innenrotation des Arms oder Beins wird vorzugsweise eine Symmetrieachse, die längs beispielsweise durch das Bein verläuft, bestimmt, um hieraus dann die entsprechende symmetrische Bewegung herzuleiten. Dabei gilt insbesondere, dass eine Außenrotation auf der einen Seite einer Außenrotation, auf der anderen Seite entspricht. Das Gleiche gilt für die Innenrotation.

Vorteilhaft werden erfindungsgemäß zweitseitige Relativlagedaten bereitgestellt, die die Lage des ersten zweitseitigen Körperteils, beispielsweise des rechten Oberschenkels relativ zum zweiten zweitseitigen Körperteil, beispielsweise dem rechten Unterschenkel beschreiben. Die Relativlagedaten beschreiben diese relative Lage vorzugsweise zumindest für zwei Relativstellungen des zweitseitigen Körperteils, die deshalb auch zweitseitige Relativstellungen genannt werden. Ein Beispiel hierfür ist volle Extension, also 0°-Flexion des rechten Beins als erste zweitseitige Relativstellung und 90°-Flexion des rechten Beins als zweite zweitseitige Relativstellung. Eine dritte zweitseitige Relativstellung wäre beispielsweise 30°-Flexion. Die Relativlagedaten können in einem Bezugssystem, das in einem ersten Körperteil ruht, beschrieben werden, wobei die Lage des zweiten Körperteils in diesem Bezugssystem beschrieben wird. Die Relativlagedaten können auch in einem anderen Bezugssystem beschrieben werden, das beispielsweise im Operationsraum ruht oder in dem Bezugssystem einer Markerdetektionseinrichtung, wie sie bei einem chirurgischen Navigationssystem (IGS, Image Guided Surgery) eingesetzt wird. Die Relativlagedaten können nicht nur die relative Lage der Körperteile sondern zumindest einen Teil der (absoluten) Lage der Körperteile in dem Bezugssystem beschreiben. Sie können insbesondere Folgendes umfassen: die erstseitigen und zweitseitigen Relativlagedaten, die Lagen der ersten und zweiten erstseitigen und zweitseitigen Körperteile für die erste und zweite zweitseitige Relativstellung und für die erste erstseitige Relativstellung und die Lage des ersten erstseitigen Körperteils in der zweiten erstseitigen Relativstellung sowie die Lage einer Körpersymmetrieebene.

Die Relativlagedaten werden vorzugsweise über Marker bestimmt, die eine vorgegebene insbesondere bekannte feste Lage zu jeweils dem ersten Körperteil und dem zweiten Körperteil haben. Bei den Markern kann es sich um aktive oder passive Marker handeln, die beispielsweise passiv elektromagnetische Wellen, insbesondere Licht, insbesondere Infrarotlicht oder Ultraschallwellen reflektieren oder derartige Wellen aussenden. Dabei ist beispielsweise eine Markereinrichtung, z.B. bestehend aus drei oder mehr Markern (z.B. ein Referenzstern) mit einem Körperteil verbunden und eine weitere Markereinrichtung mit dem anderen Körperteil verbunden. Die Verbindung kann nicht-invasiv sein, also kann beispielsweise ein flexibles Markerband mit daran angebrachten Markern um einen Körperteil gewunden werden, beispielsweise den Oberschenkel oder Unterschenkel. Alternativ oder zusätzlich können auch einzelne Marker auf den Oberschenkel aufgeklebt werden. Dies ist insbesondere dann vorzuziehen, falls es sich um gesunde, nicht zu operierende Körperteile handelt. Bei zu operierenden Körperteilen ist vorzugsweise aber nicht obligatorisch eine fest mit dem jeweiligen Knochen verbundene Markereinrichtung vorgesehen, die beispielsweise in den Knochen eingeschraubt ist. Vorzugswiese erfolgen zusätzlich Messungen von Landmarken des Körperteils, z.B. mittels Pointer oder navigiertem Instrument, die vorzugsweise ebenfalls Marker aufweisen und ebenfalls ein Beispiel für eine Markereinrichtung darstellen. An einem Pointer sind beispielsweise zwei oder mehr Marker angebracht, wobei deren relative Lage zur Pointerspitze bekannt ist.

Bei der Bestimmung der Abweichung einer Lage oder der Relativlage von einer idealsymmetrischen Situation werden vorzugsweise nicht-invasive Marker, wie Markerbänder oder einzelne, insbesondere auf der Haut aufklebbare Marker verwendet, um den Patienten so wenig wie möglich zu belasten.

Vorzugsweise werden weiter erstseitige Relativlagedaten bereitgestellt, die die Lage eines ersten erstseitigen Körperteils, also beispielsweise des linken Oberschenkels in einer ersten erstseitigen Relativstellung, also beispielsweise der vollen Extension (0°-Flexion) relativ zu einem zweiten erstseitigen Körperteil, z.B. linker Unterschenkel beschreiben. Diese Relativlagedaten werden vorzugsweise ebenfalls durch die Detektion eines Referenzsterns oder einzelner Marker (z.B. 2, 3 oder mehr) detektiert, der am ersten und/oder zweiten erstseitigen Körperteil angebracht ist, und/oder durch die Erfassung von Landmarken mittels Pointer oder eines navigierten Instruments. Die Relativlagedaten werden vorzugsweise durch Markerdetektion (Referenzstern und/oder Pointer) bestimmt, wenn eine gute Wahrscheinlichkeit vorliegt, dass die Lage der Körperteile in der ersten erstseitigen Relativstellung nicht durch die Erkrankung des Gelenks beeinträchtigt ist, also die relative Lage des ersten zweitseitigen Körperteils zum zweiten zweitseitigen Körperteils, also beispielsweise des rechten Oberschenkels zum rechten Unterschenkels, nicht durch die Erkrankung beeinträchtigt ist. Als Beispielsfall ist hier die Extension des Beins im Falle des Risses eines Kreuzbandes genannt. Der Riss des Kreuzbands beeinträchtigt die Relativlage bei Flexion, hat aber in der vollen Extension keinen Einfluss auf die relative Lage des linken Oberschenkels und linken Unterschenkels. Insbesondere wenn eine derartige verlässliche Relativstellung nicht gegeben ist, kann durch Rückgriff auf die zweitseitigen Relativlagedaten und unter Berücksichtigung der Symmetrie aus der relativen Lage der beiden zweitseitigen Körperteile in einer ersten zweitseitigen Relativstellung auf die relative Lage der erstseitigen Körperteile in der ersten erstseitigen Relativstellung geschlossen werden, ohne die relative Lage der erstseitigen Köperteile in der ersten erstseitigen Relativstellung z.B. durch Markerdetektion zu messen. Die relative Lage des ersten zum zweiten erstseitigen Körperteils, die durch die erstseitigen Relativlagedaten beschrieben wird, kann in einem beliebigen Bezugssystem beschrieben werden, beispielsweise in einem Bezugssystem, das im Operationssaal ruht, oder in dem Bezugssystem des vorgenannten Navigationssystems. Insbesondere kann die relative Lage der beiden erstseitigen Körperteile auch in einem Bezugssystem beschrieben werden, das in einem der beiden Körperteile ruht. Es wird im Folgenden angenommen, dass die erstseitigen Relativlagedaten, die durch Markerdetektion bestimmt wurden, die erste erstseitige Relativlage betreffen. Sie werden deshalb auch erste erstseitige Relativlagedaten genannt. Weiter wird angenommen, dass zweite und weitere erstseitige Relativlagedaten die zweite und weitere Relativstellungen betreffen und basierend auf den zweitseitigen Relativlagedaten (ohne Markerdetektion) berechnet werden.

Die ersten erstseitigen Relativlagedaten können also, wie oben ausgeführt, durch Detektion von Markereinrichtungen, z.B. Referenzstern und/oder Pointern bestimmt werden. Gemäß einer alternativen Ausführungsform werden aber auch die ersten erstseitigen Relativlagedaten aus den ersten zweitseitigen Relativlagedaten, die die erste zweitseitige Relativstellung betreffen, durch Symmetrieüberlegungen berechnet. Beispielsweise erfolgt eine Spiegelung der Lage des ersten und zweiten zweitseitigen Körperteils (z.B. im Bezugssystem des Navigationssystems) z.B. an der medianen Sagittalebene, um so die Lage des ersten und zweiten erstseitigen Körperteils in der ersten Relativstellung zu berechnen. Aus dieser berechneten Lage kann dann auch die relative Lage der beiden erstseitigen Körperteile in der ersten erstseitigen Relativstellung berechnet werden (siehe unten). Das Berechnungsergebnis kann dann z.B. auch mit der detektierten Lage des ersten und/oder zweiten erstseitigen Körperteils verglichen werden. Dadurch können Abweichungen der Anordnung von Körperteilen von der idealsymmetrischen Anordnung erkannt werden. Erfindungsgemäß kann dies auch für eine einzige Relativstellung durchgeführt werden, so dass bei dieser Variante der Erfindung, die auf die zweite Relativstellung bezogenen Berechnungen nicht notwendig sind. Weiter können erfindungsgemäß berechnete Relativstellungen, genauer die (beispielsweise in der oben beschriebenen Art) berechnete relative Lage der erstseitigen Körperteile in der zweiten erstseitigen Relativstellung mit detektierten Lagen der erstseitigen Körperteile in der zweiten erstseitigen Relativstellungen verglichen werden. Dadurch können Abweichungen der erstseitigen Relativstellungen von den zweitseitigen Relativstellungen, die insbesondere auf eine Abweichung der Kinematik des erstseitigen Gelenks vom zweitseitigen Gelenk zurückzuführen ist, erkannt werden. Dies kann insbesondere genutzt werden, um krankhafte Gelenke zu erkennen und/oder ein Operationsergebnis zu überprüfen.

Vorzugsweise ist die erste erstseitige Relativstellung zur ersten zweitseitigen Relativstellung symmetrisch, und die zweite erstseitige Relativstellung ist symmetrisch zur zweiten zweitseitigen Relativstellung. Mit den jeweiligen Relativstellungen sind Lagen der Körperteile verknüpft. Diese Lagen werden vorzugsweise für die erste erstseitige Relativstellung, die erste zweitseitige Relativstellung und die zweite zweitseitige Relativstellung mittels einer Detektionseinrichtung bestimmt, beruhen also auf Detektionssignaldaten.

Vorzugsweise wird erfindungsgemäß aus den erstseitigen und zweitseitigen Relativlagedaten die relative Lage des ersten erstseitigen Körperteils zum zweiten erstseitigen Körperteil in der zweiten erstseitigen Relativstellung berechnet. Beispielsweise wird also die relative Lage des linken Ober- und Unterschenkels bei 90°-Flexion aus der relativen Lage des linken Ober- und Unterschenkels bei 0°-Flexion (voller Extension) und aus der relativen Lage des rechten Ober- und Unterschenkels bei 0°-Flexion und 90°-Flexion berechnet.

Beispielsweise wird aus den zweitseitigen Relativlagedaten ein Schwenkwinkel (z.B. 90°) bestimmt, um den das zweite zweitseitige Körperteil (z.B. rechter Unterschenkel) relativ zum ersten zweitseitigen Körperteil (z.B. rechtem Oberschenkel) beim Übergang von der ersten zweitseitigen Relativstellung (0°-Flexion) zur zweiten zweitseitigen Relativstellung (90°-Flexion) schwenkt. Vorzugsweise wird dann das zweite erstseitige Körperteil (z.B. linker Unterschenkel) relativ zum ersten erstseitigen Körperteil (z.B. linkem Oberschenkel) ausgehend von der ersten Relativstellung (z.B. 0°-Flexion) um denselben Schwenkwinkel (z.B. 90°) verschwenkt, um so die Lage des zweiten erstseitigen Körperteils (z.B. linken Unterschenkel) in der zweiten erstseitigen Relativstellung (90°-Flexion) zu berechnen.

Bei Bestimmung der zweiten erstseitigen Relativstellung werden vorzugsweise Symmetrieregeln berücksichtigt, die die kinematische Symmetrie der Gelenkbewegung beschreiben. Beispielsweise erfolgt hierzu eine Spiegelung zumindest eines zweitseitigen Relativvektors an einer Körpersymmetrieebene, insbesondere an der medianen Sagittalebene. Beispielsweise wird zumindest ein zweitseitiger Relativvektor, der den Übergang von der ersten zweitseitigen Relativstellung zur zweiten zweitseitigen Relativstellung beschreibt, in Komponenten zerlegt, für die jeweils eine Symmetrieregel angewendet wird. Beispielsweise kann der Vektor eine Komponente in einer Ebene aufweisen, die parallel zu der medianen Sagittalebene ist, die als Parallelkomponente bezeichnet wird, und eine Komponente aufweisen, die senkrecht zur medianen Sagittalebene ist und die als senkrechte Komponente bezeichnet wird. Auf die parallele Komponente kann die Symmetrieregel angewendet werden, dass sie ungespiegelt für einen erstseitigen Relativvektor übernommen wird. Für die senkrechte Komponente kann die Symmetrieregel angewendet werden, dass sie an der medianen Sagittalebene gespiegelt wird. Addiert man die parallele Komponente und die gespiegelte senkrechte Komponente, so erhält man den erstseitigen Relativvektor, der den Übergang von der ersten erstseitigen Relativstellung zur zweiten erstseitigen Relativstellung beschreibt. Vorzugsweise werden zwei zweitseitige Relativvektoren für das erste zweitseitige Körperteil bestimmt, die beispielsweise an definierten Stellen, z.B. an zwei durch Landmarken definierten Punkten des Körperteils ansetzen. Aus diesen werden dann dazu symmetrische erstseitige Relativvektoren bestimmt, die an den entsprechenden Stellen des ersten erstseitigen Körperteils ansetzen und den Übergang beschreiben.

Weiter wurden die erstseitigen und zweitseitigen Relativlagedaten vorzugsweise in einer Lage gewonnen oder für eine Lage bestimmt, in der das erste erstseitige Körperteil und das erste zweitseitige Körperteil symmetrisch zueinander bezüglich der medianen Sagittalebene angeordnet sind. Vorzugsweise basierend auf dieser Voraussetzung oder Annahme werden dann die erstseitigen und zweitseitigen Relativvektoren bestimmt und/oder angewendet.

Die Bestimmung oder Bereitstellung der Lage der medianen Sagittalebene ist nicht obligatorisch. So kann man den Berechnungen auch ohne Bestimmung der medianen Sagittalebene die Annahme zu Grunde legen, dass die Bewegung nur in einer Ebene erfolgt, die parallel zur medianen Sagittalebene ist, somit also keine senkrechte Komponente existiert.

Ist die Lage der medianen Sagittalebene nicht bekannt (nicht bereitgestellt), so kann die mediane Sagittalebene beispielsweise mittels Pointer bestimmt werden, indem Landmarken des Körpers abgegriffen werden, die typischer Weise symmetrisch zu der medianen Sagittalebene sind. Verbindet man diese Landmarken durch eine gerade Linie und legt auf halber Strecke eine zu der Linie senkrechte Ebene, so kann man die so erhaltene Ebene als mediane Sagittalebene definieren. Befindet sich beispielsweise das erste erstseitige Körperteil und das erste zweitseitige Körperteil in einer definierten (beispielsweise 0°-Flexion) und zueinander parallelen Stellung, können auch die Lagedaten für das erste erstseitige Körperteil und das erste zweitseitige Körperteil verwendet werden, um so über eine virtuelle Verbindung der Körperteile und durch Bestimmen einer zur Verbindung senkrechten Ebene die mediane Sagittalebene zu bestimmen.

Insbesondere können Bereiche bestimmt werden, die in Abhängigkeit von der Relativstellung des ersten erstseitigen Körperteils zum zweiten erstseitigen Körperteil hinsichtlich ihrer relativen Lage eine bestimmte Eigenschaft erfüllen. Insbesondere kann bestimmt werden, welche relative Lage für einen ersten zweitseitigen Bereich zum zweiten zweitseitigen Bereich gilt, wenn das zweitseitige Gelenk beispielsweise eine bestimmte Bewegung durchführt. Auf diese Art und Weise kann eine Soll-Funktion für die relative Lage der Bereiche festgelegt werden. Diese Soll-Funktion kann dann mit der auf der ersten Seite gegebenen Funktion verglichen werden, um so beispielsweise festzustellen, ob das zweitseitige Gelenk gesund ist.

Beispiele für Bereiche sind Punkte oder Bereiche auf der Oberfläche des Körperteils oder Vertiefungen oder Bohrungen in dem Körperteil. Einen Hinweis auf die relative Lage zwischen dem ersten Bereich und dem zweiten Bereich gibt eine Relativgröße. Die Berechnung der Relativgröße hängt von der relative Lage des ersten Bereichs zum zweiten Bereich ab. Die beschreibt insbesondere eine geometrische Beziehung zwischen den Bereichen. Beispiele für Relativgrößen sind eine Winkelbeziehung zwischen Geraden oder Achsen, die durch die Bereiche und/oder Körperteile laufen oder der relative Abstand zwischen den Bereichen oder ein Vektor, der die beiden Bereiche verbindet. Insbesondere befindet sich einer der Bereiche auf einem ersten einseitigen Körperteil und der andere Bereich auf einem zweiten erstseitigen Körperteil.

Die Lage der Bereiche können in einem allgemeinen Bezugssystem, z.B. dem Bezugssystem des Navigationssystems oder des Operationssaals angegeben werden. Sie können aber z.B. auch im Bezugssystem des Körperteils angegeben werden, in dem der Bereich ruht. Insbesondere erfolgt die Berechnung der Relativgrößen unter Einbeziehung der Daten über die relative Lage zwischen den beiden Körperteilen. Diese Berechnung erfolgt vorzugsweise für die verschiedenen Relativstellungen, um so bestimmen zu können, wie sich die Relativgröße in Abhängigkeit von der Relativstellung ändert.

Die Bereichsdaten, die die relative Lage der Bereiche in der ersten und/oder zweiten Relativstellung beschreiben, können beispielsweise mittels Pointer gewonnen werden, die in der ersten und/oder zweiten Relativstellung mit dem jeweiligen Bereich in Kontakt gebracht werden. Die Pointer umfassen typischerweise zumindest zwei Marker. Durch Detektion der Marker und aufgrund der bekannten relativen Lage zwischen den Markern und der Pointerspitze lässt sich dann die Lage des Bereichs, der mit der Pointerspitze in Kontakt ist, z.B. im Bezugssystem des Körperteils, in dem sich der Bereich befindet, oder des Navigationssystems bestimmen. Basierend hierauf wird dann die relative Lage zwischen den Bereichen berechnet, um so die Bereichsdaten zu bestimmen.

Bei einer Ausführungsform der Erfindung, ist es nicht erforderlich, dass die Körperteile die erste und/oder zweite erstseitige Relativstellung einnehmen, um die Bereichsdaten zu erfassen. Sie können auch in einer weiteren Relativstellung erfasst werden, die nicht mit der ersten oder zweiten Relativstellung übereinstimmt. Für diese weitere Relativstellung ist aber vorzugsweise ebenfalls die relative Lage des ersten und zweiten erstseitigen Körperteils zu ihrer Lage in der ersten und/oder zweiten Relativstellung bekannt. Basierend hierauf kann dann durch das erfindungsgemäße Verfahren berechnet werden, wo sich der Bereich in der ersten und/oder zweiten Relativstellung befinden würde. Auf diese Weise erhält man dann ebenfalls die Bereichsdaten für die erste und/oder zweite Relativstellung.

Auch können die Bereichsdaten zeitaktuell verarbeitet werden, und es kann überprüft werden, ob diese eine vorgegebene Funktion erfüllen. Also beispielsweise kann basierend auf den aktuellen Bereichsdaten, die sich beispielsweise aus der aktuellen Position eines Pointers ergeben, berechnet werden, ob sich die Relativgröße bei einem Übergang von der ersten zur zweiten Relativstellung verändert, oder ob sie gleich bleibt. Dazu genügt es, in irgendeiner erstseitigen Relativstellung die Lage eines der beiden Bereiche an einem der beiden erstseitigen Körperteile z.B. mit einem Pointer zu erfassen. Dann wird der Bereich an dem anderen der beiden Körperteile z.B. mittels eines Pointers erfasst, und es wird zeitaktuell berechnet, ob eine vorgegebene Bedingung erfüllt ist oder nicht. Die Bedingung kann beispielsweise sein, dass sich die Relativgröße bei einem Übergang von der ersten zur zweiten Relativstellung nicht ändert. Der Pointer kann dann verfahren werden, um so einen der beiden Bereiche zu ändern. Vorzugsweise wird für jede Pointerstellung zeitaktuell bestimmt, ob die Bedingung erfüllt ist. Insbesondere wird dies angezeigt. Beispielsweise erfolgt eine Anzeige, dass in der aktuellen Stellung des Pointers die vorgegebene Bedingung erfüllt ist. Der Operateur weiß dann, dass er einen passenden Bereich gefunden hat. Dies ist erfindungsgemäß möglich, ohne die Körperteile relativ zueinander zu bewegen. Insbesondere müssen die Körperteile nicht bewegt werden, um die erste und zweite erstseitige Relativstellung einzunehmen, sondern können eine beliebige andere erstseitige Relativstellung einnehmen, bei der das Einführen des Pointers beispielsweise am einfachsten für den Operateur ist. Die Bewegung in die erste und zweite erstseitige Relativstellung und die Erfassung der Bereiche in diesen Stellungen erfolgt dann durch Berechnung, also virtuell. Gemäß einer Ausführungsform werden die verschiedenen Relativstellungen verwendet, um einen Bewegungsablauf beispielsweise auf der zweiten Seite zu bestimmen. Dieser Bewegungsablauf kann dann durch das erfindungsgemäße Verfahren und insbesondere unter Beachtung von Symmetrieregeln, in einen Soll-Bewegungsablauf für die erste Seite umgerechnet werden. Insbesondere kann durch Messung mehrerer Relativstellungen der ersten Seite ebenfalls ein Bewegungsablauf (Ist-Bewegungsablauf) für die erste Seite bestimmt werden_{,} und dieser Ist-Bewegungsablauf kann mit dem durch die zweitseitigen Relativstellungen bestimmten Soll-Bewegungsablauf verglichen werden. Insbesondere kann angezeigt werden, falls es zu einer Abweichung zwischen dem Soll- und dem Ist-Bewegungsablauf kommt.

Vorteilhaft werden zur Bestimmung der zweiseitigen Relativlagedaten Markereinrichtungen verwendet, die insbesondere nicht an einem Knochen befestigt sind, sondern bevorzugt die Haut des Körperteiles nicht oder nicht wesentlich durchdringen. Vorzugsweise wird beispielsweise eine Markereinrichtung verwendet, die kraft- und/oder formschlüssig um das Körperteil herum gebunden werden kann, also beispielsweise ein Markerband (zum Beispiel so genanntes Headband). Vorzugsweise durchdringen die Markereinrichtungen also nicht das Körperteil, sondern halten bevorzugt durch Kontakt mit der Oberfläche eine feste relative Lage relativ zum Körperteil ein.

Die Erfindung ist auch auf ein Programm gerichtet, das das vorbestimmte Verfahren insbesondere mit einer Datenverarbeitungseinrichtung, beispielsweise mit einem Computer durchführt. Weiter ist sie auf ein Speichermedium, wie z.B. ROM oder CD gerichtet, das das Programm speichert sowie auf eine Signalwelle, die beispielsweise beim Internet-Downloadvorgang die das Programm darstellende Information überträgt.

Die Erfindung betrifft weiter ein Navigationssystem, das eine Detektionseinrichtung aufweist, um Marker von Markereinrichtungen, z.B. Referenzsterne oder Pointer zu detektieren, um so die Lage der Körperteile und/oder von Bereichen der Körperteile zu erfassen. Die Detektionseinrichtung erzeugt Detektionssignale, die von der Datenverarbeitungsanlage umgesetzt werden, um die Relativlagedaten und/oder Bereichsdaten zu bestimmen, die dann die Grundlage für die weitere Berechung gemäß den erfindungsgemäßen Verfahren bilden.
Figur 1 zeigt einen Femur und eine Tibia mit Kreuzbandersatz.
Figur 2 zeigt schematisch ein linkes und rechtes Bein von oben sowie ein Navigationssystem, das an den Beinen angebrachte Referenzsterne detektiert.
Figur 3 zeigt die Bestimmung von Relativvektoren für zwei Relativstellungen.
Figur 4 zeigt die Anwendung der bestimmten Relativvektoren zur Berechnung der relativen Lage des linken Unterschenkels.
Figur 5 zeigt die Anwendung der Erfindung auf den linken und rechten Arm.
Figur 6 zeigt einen Fall, bei dem eine ideale Symmetrie nicht gegeben ist.
Figure 7 zeigt die Situation aus Figur 6, mit gespiegeltem Femur und gespiegelter Tibia.

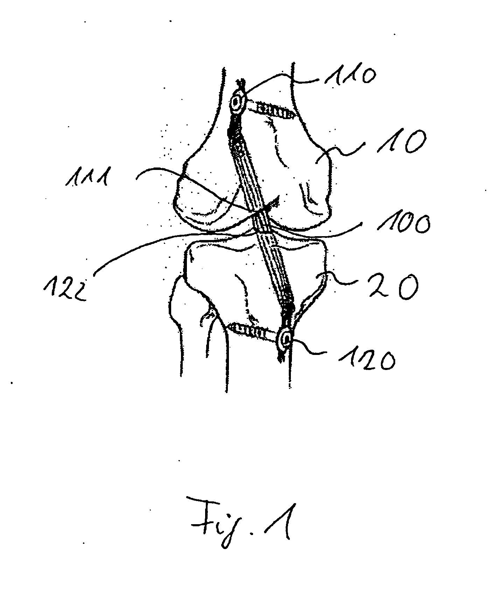

Figur 1 zeigt das gelenkseitige Ende des Femurs 10 und der Tibia 20. Als Kreuzbandersatz wird beispielsweise eine Patellasehne oder ein Implantatband 100 verwendet, das an seinen Enden über Schrauben 110 und 120 jeweils am Femur und an der Tibia befestigt ist. Das Band 100 läuft durch einen Bohrkanal im Femur 10 und in der Tibia 20. Diese Bohrkanäle haben gelenkseitige Austrittsöffnungen 111 (am Femur) und 122 (an der Tibia). Um die Funktion des Kreuzbandes gut ersetzen zu können, ist es wünschenswert, dass der relative Abstand zwischen den Austrittsöffnungen 111 und 122 unabhängig vom Grad der Flexion möglichst konstant bleibt Diese Austrittsöffnungen 111 und 122 stellen Beispiele für die vorgenannten Bereiche dar, für die die Relativgröße (der Abstand) unabhängig von den Relativstellungen möglichst unverändert sein soll.

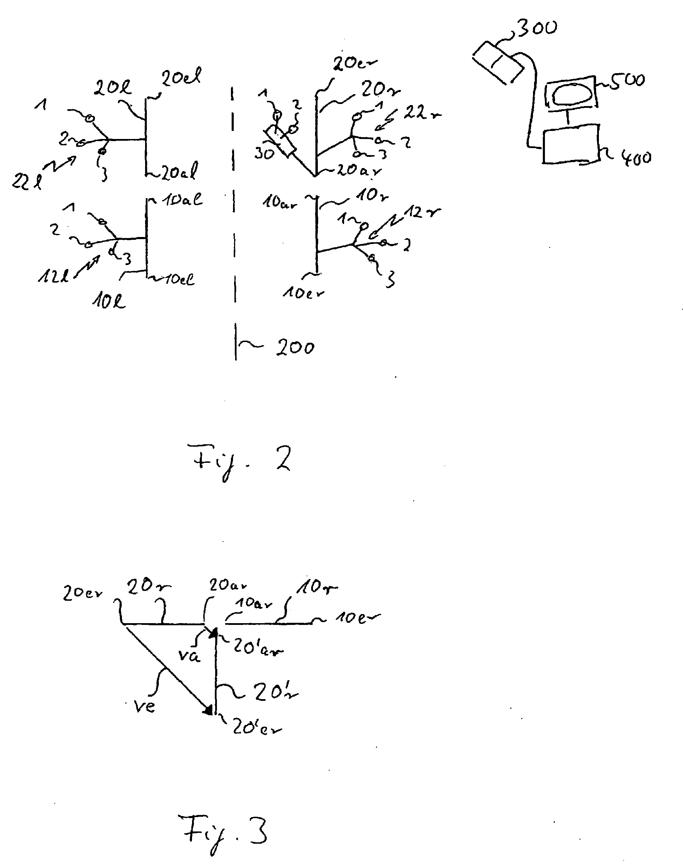

Figur 2 zeigt schematisch von oben betrachtet das linke und rechte Bein eines liegenden Menschen. Der linke Femur ist mit 101 bezeichnet, die linke Tibia mit 201. Der rechte Femur ist mit 10r bezeichnet, die rechte Tibia mit 20r. Gestrichelt ist gezeichnet die mediane Sagittalebene 200. Das rechte und linke Bein sind symmetrisch bzgl. dieser Ebene angeordnet. An jedem Bein befinden sich Markereinrichtungen 221, 121, 22r und 12r jeweils mit Markern 1, 2 und 3, die durch die Detektionseinrichtung 300 detektiert werden. Es wird angenommen, dass das linke Kniegelenk krank ist, also beispielsweise einen Kreuzbandriss hat und somit einer Operation unterzogen wird. In diesem Fall sind die Markeinrichtungen (Referenzsteme 221 und 121) vorzugsweise fest mit dem Femur und der Tibia verbunden. Am gesunden rechten Bein sind die Markereinrichtungen (z.B. "ENT-Headband 12r und 22r) vorzugsweise nicht invasiv angebracht, sondern beispielsweise mittels eines flexiblen Bandes jeweils um den Femur und die Tibia gewunden. Alternativ oder zusätzlich können auch einzelne Marker auf den Femur oder die Tibia geklebt werden. Hierbei können von Kopfoperationen bekannte, so genannte Headbands verwendet werden.

Vorzugsweise werden Bezugssysteme dem Femur 101 und 10r und der Tibia 201 und 20r zugeordnet. Hierzu wird vorzugsweise mittels eines Pointers 30 an einer gelenkseitigen Landmarke, vorzugsweise dem Tibiaplateau, diese Landmarke, also das Tibiaplateau von einem Navigationssystem erfasst, das die Marker am Pointer detektiert und somit die Lage der Pointerspitze erfasst. Als Richtung des Pointers 30 wird vorzugsweise bei der Erfassung die anterior-posterior-Richtung gewählt, so dass auch diese Richtung vom Navigationssystem miterfasst wird. Als weiteres wird dann vorzugsweise der Pointer 30 auf halber Höhe der Tibia an der am weitesten anterior gelegenen Stelle aufgesetzt, um diese Landmarke zu erfassen. Diese Landmarke wird dann von der Datenverarbeitungseinrichtung des Navigationssystems in die bereits erfasste anterior-posterior-Richtung verschoben, bis eine Linie geschnitten wird, die von der bereits erfassten Tibia-Plateau-Landmarke ausgeht und senkrecht zu der anterior-posterior-Richtung ist. Dieser Schnittpunkt definiert dann mit der Tibia-Plateau-Landmarke die Richtung der Tibia-Achse. Diese Tibia-Achse kann dann eine der Achsen des mit der Tibia verbundenen Koordinatensystems bilden. Die anderen beiden Achsen sind beispielsweise senkrecht dazu, wobei eine beispielsweise in anterior-posterior-Richtung zeigen kann. Die in Figur 2 gezeigten Linien 201 und 20r können als Abschnitte der Tibia-Achse aufgefasst werden, die beispielsweise von dem Tibia-Plateaupunkt, der z.B. mit dem Endpunkt 20ar übereinstimmt, ausgehen und sich über eine vorgegebene Länge z.B. von einem Punkt 20ar zu einem Punkt 20er erstrecken, wobei der Punkt 20ar das dem Gelenk zugewandte Ende bezeichnet. Entsprechend gibt es Endpunkte 20al und 20el an der linken Tibia-Achse 201. Somit ist auch die Lage der Endpunkte im jeweiligen Tibia-Bezugssystem bekannt.

Durch den oben beschriebenen Registrierungsvorgang ist die relative Lage des Tibia-Bezugssystems zu den Referenzsternen 22r und 221 und somit zu deren Markern jeweils bekannt, und die jeweiligen Bezugssysteme sind somit im Bezugssystem des Navigationssystems registriert.

Als nächster Schritt sind dann noch die Bezugssysteme des Femurs jeweils zu bestimmen. Hierzu wird beispielsweise wie folgt vorgegangen. Das mit der Tibia verbundene Bezugssystem wird kopiert und entlang der Tibia-Achse, insbesondere um eine vorbestimmte Länge (beispielsweise unter Bezugnahme auf den Punkt 20ar oder 20al) in Richtung des Femurs verschoben, so dass der Ursprung des kopierten Bezugssystems im Femur liegt. Das so erhabene kopierte Bezugssystem wird dann zum Bezugssystem des Femurs. Insbesondere können die in Figur 2 gezeigten Abschnitte 10r und 101 Bestandteil einer Koordinatenachse des Femur-Bezugssystems sein, die bei Extension des Beins Teil einer verlängerten Tibia-Achse ist. Alternativ kann ein Femur-Bezugssystem auch durch Erfassen von Landmarkem am Femur festgelegt werden.

Auf die oben genannte Weise sind nun die jeweiligen Achsenabschnitte 201, 20r, 10r und 101 im Bezugssystem des Navigationssystems registriert. Insbesondere ist die relative Lage der Achsabschnitte zueinander bekannt und insbesondere können auch Endpunkte 10ar, 10er und 20ar und 20er des Femur-Abschnittes bestimmt werden, wobei beispielsweise festgelegt wird, dass im Extensionszustand ein vorbestimmter Abstand zwischen 20ar und 10ar und/oder zwischen 20er und 10er besteht. Weiter kann festgestellt werden, dass ein vorbestimmter Abstand zwischen 10ar und 10per sowie zwischen 20ar und 20er besteht. Somit ist die relative Lage der Achsabschnitte und auch der Endpunkte durch Detektion der Markereinrichtungen 221, 121, 22r und 12r bestimmbar. Aus diesen Detektionssignaldaten können somit die Relativlagedaten für die rechte und die linke Seite abgeleitet werden.

Figur 3 zeigt den Übergang von 0°-Flexion zu 90°-Flexion auf der rechten Seite. Die Achsabschnitte für 0°-Flexion (Extension) sind mit 10r und 20r bezeichnet. Für 90°-Flexion sind die Achsabschnitte mit 10r und 20'r bezeichnet. Die relative Lage zwischen 10r und 20'r wird mittels Markereinrichtungen detektiert, und die Detektionssignale werden beispielsweise einer Datenverarbeitungseinrichtung 400 (siehe Figur 2) zugeführt. Die relative Lage zwischen 10r und 20r ist bereits bekannt, wie oben beschrieben wurde. Entsprechend können auch die Lage der Endpunkte 20'ar und 20'er bestimmt werden. Insgesamt lassen sich somit aus den vorhandenen Daten Relativvektoren va und ve bestimmen. Der Relativvektor va zeigt vom Endpunkt 20ar zum Endpunkt 20'ar. Der Relativvektor ve zeigt vom Endpunkt 20er zum Endpunkt 20'er. Dies stellt nur ein Beispiel dar. Eine andere Vorgehensweise wäre, beispielsweise Relativvektoren von den Endpunkten 10ar zum Endpunkt 20'ar zu bestimmen und vom Endpunkt 10er zum Endpunkt 20'er. Weiter könnte die Änderung der Lage auch über Winkel beschrieben werden, beispielsweise über den 90°-Winkel und durch die Ebene, in der die Streckenabschnitte 20r, 20'r und 10r liegen. Im Folgenden wird die Erfindung beispielhaft mit Hilfe der vorgenannten Vektoren va und ve beschrieben.

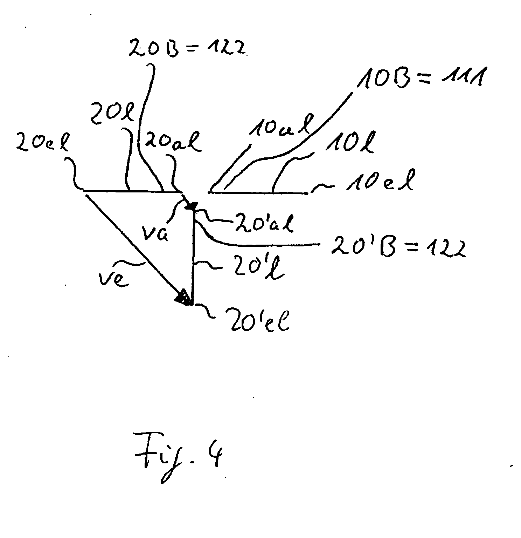

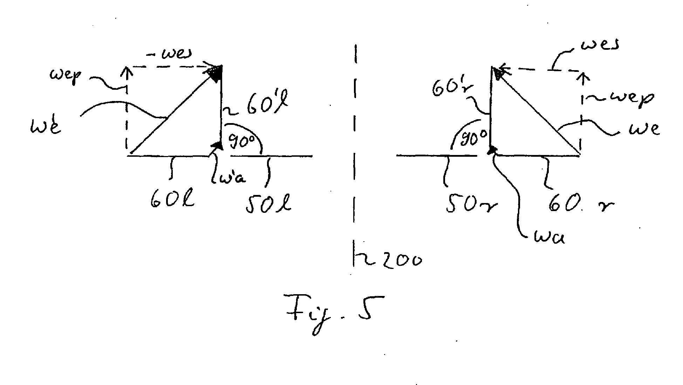

Wie in Figur 4 gezeigt ist, ist in der bekannten Ausgangsstellung das Bein in Extension, d.h. die Lage des Beins wird durch die Streckenabschnitte 101 und 201 beschrieben. Um nun zu berechnen, wie die relative Lage des linken Beins bei 90°-Flexion wäre, ohne das linke Bein tatsächlich in 90°-Flexion zu bewegen, werden die vorgenannten, bereits berechneten Vektoren va und ve verwendet. Der Vektor va wird an den Endpunkt 20al angesetzt, um zum Endpunkt 20'al zu zeigen. Der Vektor ve wird an den Endpunkt 20el angesetzt, um zu dem Endpunkt 20'el zu zeigen. Dies bedeutet, dass man davon ausgeht, dass die linke Tibia die selbe Relativbewegung zum linken Femur durchführt, wie die rechte Tibia relativ zum rechten Femur. Im vorgenannten Beispiel wurde davon ausgegangen, dass die Bewegung in einer Ebene erfolgt, die parallel zur medianen Sagittalebene ist. Die Bewegung kann natürlich auch Anteile enthalten, die von dieser exakten Parallelität abweichen. Dieser Fall kann durch die Berücksichtigung von Symmetrieregeln erfindungsgemäß behandelt werden. Dies wird im Folgenden anhand eines Beispieles, wie es in Figur 5 gezeigt ist, erläutert.

Figur 5 soll beispielsweise ein Blick von oben auf einen stehenden Patienten sein, der seinen rechten und linken Arm gehoben hat. 501 bezeichnet den dorsal senkrecht zur medianen Sagittalebene ausgestreckten linken Oberarm und 50r den dorsal senkrecht zur medianen Sagittalebene 200 ausgestreckten rechten Oberarm. Der linke Unterarm 601 befindet sich in Verlängerung zu dem linken Oberarm 501 und der rechte Unterarm 60r befindet sich in Verlängerung zum rechten Oberarm 50r. Die Relativstellungen 501 zu 601 und 50r zu 60r werden jeweils wiederum mit Markereinrichtungen erfasst und die Achsabschnitte 501 zu 601 sowie 50r zu 60r werden jeweils wiederum mit Pointer erfasst, so dass sie analog zu dem in Figur 2 beschriebenen Verfahren, in einem Bezugssystem, insbesondere im Bezugssystem des Navigationssystems bekannt sind. Wiederum wird angenommen, dass die rechte Seite die gesunde Seite ist, also das rechte Ellbogengelenk gesund ist, während das linke Ellbogengelenk krank ist. Während 50r und 60r zusammen die erste zweitseitige Relativstellung bilden, bilden 50r und 60'r die zweite zweitseitige Relativstellung. Für diese wird ebenfalls wieder die relative Lage zwischen 50r und 60'r mittels Markerdetektion bestimmt. Insbesondere können auch die relativen Lagen zwischen Endpunkten der Streckabschnitte 50r und 60'r bestimmt werden. Durch den Vergleich der Lage dieser Endpunkte können Relativvektoren we und wa bestimmt werden, in analoger Weise wie in Figur 3. Diese Relativvektoren können nun zerlegt werden in Anteile parallel zur medianen Sagittalebene und senkrecht dazu. Der parallele Anteil von we wird mit wep bezeichnet und der senkrechte mit wes. Die senkrechten Anteile sind parallel zur Frontalebene. Wie oben beschrieben wurde, wird davon ausgegangen, dass die erste zweitseitige Relativlage mit den Achsabschnitten 50r und 60r bzgl. der medianen Sagittalebene 200 symmetrisch zu der ersten erstseitigen Relativposition ist, die durch 501 und 601 beschrieben wird. Unter dieser Voraussetzung wird aus dem Vektor we und insbesondere aus den Komponenten wes und wep der Relativvektor w'e für die linke Seite bestimmt, der sich aus wep-wes berechnet. Somit ergibt sich, dass der resultierende Vektor w'e bzgl. der medianen Sagittalebene symmetrisch zu dem Vektor we ist. Entsprechend wird auch ein Vektor w'a berechnet, der symmetrisch zum Vektor wa ist. Der symmetrische Vektor w'e wird dann an das Ende des Achsabschnittes 601 angesetzt, das dem Ellbogengelenk abgewandt ist, und der Relativvektor w'a wird an das Ende des Achsabschnittes 601 angesetzt, das dem Ellbogengelenk zugewandt ist. Die Spitzen der beiden Vektoren zeigen dann auf die jeweiligen Enden des (um 90°) geschwenkten Achsenabschnittes 601, so dass sich die Lage des Achsabschnittes 601 ergibt, wobei die Symmetrieregeln beachtet wurden. Die Lage des Achsabschnittes 601 bezeichnet somit die Lage des linken Unterarms unter der Annahme, dass dieser symmetrisch zum rechten Unterarm bewegt wird und somit ebenfalls um 90° gebeugt bzw. geschwenkt wird. Es kann also bestimmt werden, wie der linke Unterarm bei 90°-Beugung liegen würde, wenn das linke Ellbogengelenk eine kinematische Symmetrie zum rechten Ellbogengelenk aufweisen würde und sich ebenfalls wie ein gesundes Gelenk verhalten würde.

Neben den oben genannten Beispielen von 90°-Flexion sind natürlich auch andere Flexionsgrade wie insbesondere 30°, 20° oder 60° möglich. Die oben beschriebene Weise, die Änderung der Relativlage mittels Vektoren zu beschreiben, kann mathematisch auch anders erfolgen, wie beispielsweise unter Verwendung von Winkeln und Ebenen, in denen die Bewegung stattfinden soll.

Die oben genannten Relativlagedaten wurden insbesondere unter der Annahme der Berechnung zugrunde gelegt, dass sie in Neutralposition der jeweiligen Körperteile gewonnen wurden. Für das Bein gilt beispielsweise, dass in Extension eine Verschraubung der Tibia relativ zum Femur stattfindet, die ein geringes Spiel einer relativen Rotation der Tibia relativ zum Femur zulässt. In anderen Relativlagen, beispielsweise 30°-Flexion oder 90°-Flexion ist diese Verschraubung nicht gegeben. Dies gilt insbesondere für das kranke Bein (Kreuzbandriss), weshalb die erfindungsgemäße virtuelle Beugung des kranken Beines als vorteilhaft angesehen wird. Für das gesunde Bein wird zur Festlegung der zweiten Relativstellung oder weiteren Relativstellungen, also zur Festlegung der jeweiligen Neutralstellung die Tibia relativ zum Femur rotiert, und als Neutralstellung wird der Mittelwert der beiden extremen Rotationswinkel (maximale Innenrotation und maximale Außenrotation) gewählt. Wie bereits ausgeführt, ist dies beim kranken Bein, insbesondere beim Riss des Kreuzbandes nicht möglich, weil hier das Kreuzband nicht mehr die Rotationswinkel für Innen- und Außenrotation begrenzt.

Um Bereiche zu finden, die eine bestimmte Bedingung erfühlen, also für die eine Relativgröße beispielsweise konstant ist, kann wie im Folgenden beschrieben vorgegangen werden. Beispielsweise wird mit einem Pointer ein bestimmter Bereiche 20'B bezeichnet (siehe Figur 4), der sich beispielsweise als gelenkseitiges Ende einer Bohrung durch die Tibia eignet, um dadurch ein Band 100 (siehe Figur 1) zu führen. Weiter wird z.B. mittels eines Pointers ein Bereich 10B (siehe Figur 4) bestimmt, der sich beispielsweise ebenfalls in der Nähe des gelenkseitigen Endes des Femurs befindet. In dem in Figur 4 gezeigten Beispiel liegen die Bereiche 20'B und 10B auf den jeweiligen Achsabschnitten. Dies ist rein beispielhaft. Im Praxisfall können sie durchaus außerhalb des Achsabschnittes liegen. Ein Beispiel für den Bereich 20'B ist der in Figur 1 gezeigte Bereich 122. Entsprechendes wurde in Figur 4 vermerkt. Ein Beispiel für den Bereich 10B ist der Bereich 111 in Figur 1.

Durch den Pointer ist die Relativlage der Bereiche 20'B und 10B relativ zu dem Koordinatensystem der Tibia und des Femurs bekannt. Insbesondere sind beispielsweise auch Relativlagen zu den Endpunkten 20'al und 20'el sowie 10al und 10el bekannt.

Erfindungsgemäß kann nun rein virtuell das Bein bewegt werden. Beispielsweise wird die Tibia von der mit 20'1 bezeichneten Position in die Position 201 bewegt (siehe Figur 4). Auch können andere Zwischenpositionen dabei eingenommen werden. Insgesamt ergeben sich somit mindestens zwei Relativstellungen, für die die relative Lage zwischen dem Bereich 20'B und 10B berechnet werden kann. Insbesondere kann überprüft werden, ob der Abstand für die verschiedenen Relativstellungen gleich ist oder sich ändert. Verändert sich der Abstand, so kann dies angezeigt werden und ein Operateur kann dann beispielsweise den Pointer verfahren, um einen neuen Bereich am Femur zu finden, der beispielsweise ebenfalls in der Nähe des Gelenks ist und der die gewünschte Bedingung erfüllt.

Wie oben erwähnt wurde, ist die Position des linken kranken Beins bei 90°-Flexion nicht zuverlässig. Es kann aber vorteilhaft sein, in dieser Stellung zwischen dem Gelenk liegende Bereiche mit dem Pointer abzugreifen, da man bei 90°-Flexion besser zwischen Femur und Tibia mit dem Pointer hineingelangt. Um dennoch eine definierte Stellung bei 90°-Flexion für die Tibia zu haben, kann mittels der an der Tibia angebrachten Markereinrichtung überprüft werden, ob diese 90°-Flexionsstellung mit der errechneten 90°-Flexionsstellung (Neutralstellung bei 90°-Flexion) übereinstimmt. Ist diese Übereinstimmung gegeben, so dann dies angezeigt werden, und der Operateur kann dann in dieser als Neutralstellung erkannten 90°-Flexionsstellung die Bereiche mit dem Pointer abgreifen. Alternativ oder zusätzlich kann durch Detektion der an der Tibia angebrachten Markereinrichtung berechnet werden, wo der mit dem Pointer abgegriffene Bereich liegen würde, wenn die Tibia eine errechnete Relativstellung und/oder die erste erstseitige Relativstellung einnehmen würde. Auf diese Art und Weise kann für jede Position des Pointers berechnet werden, ob der Abstand zwischen 20'B und 10B gleich dem Abstand zwischen 20B und 10B ist, ohne das Bein zu bewegen. Natürlich kann dies noch für mehrere Relativstellungen berechnet werden. Der Pointer wird beispielsweise an verschiedene Stellen 10B gebracht, und das Navigationssystem 300, 400 und 500 zeigt mit der Anzeige 500 an, falls der Abstand gleich ist oder z.B. um weniger als einen vorbestimmten Prozentsatz für die verschiedenen berechneten Relativstellungen abweicht.

Die vorliegende Erfindung eignet sich auch zur Überprüfung der Bewegung und von Relativstellungen eines kranken Gelenks durch Vergleich mit Bewegungen und Relativstellungen des gesunden Gelenks. Insbesondere können mehrere Relativstellungen für das gesunde Gelenk detektiert und gespeichert werden und hieraus ein Bewegungsablauf errechnet werden. Mit dem erfindungsgemäßen Verfahren können dann entsprechende (kinematisch symmetrische) Relativstellungen und Bewegungsabläufe für die Körperseite mit dem kranken Gelenk berechnet werden. Anhand der an der kranken Seite angebrachten Markereinrichtungen (invasiv oder auch nicht invasiv) kann dann überprüft werden, ob der Bewegungsablauf kinematisch symmetrisch zu dem gesunden Gelenk ist oder ob eine kinematisch symmetrische Stellung eingenommen wurde. Insbesondere kann somit auch erkannt werden, ob der Bewegungsablauf einem gesunden Ablauf entspricht. Beispielsweise können somit auch Kreuzbandrisse erkannt werden.

Neben der oben beschriebenen medianen Sagittalebene 200 können auch noch andere Symmetrieebenen oder Symmetrieachsen bei der Bestimmung der zweiten erstseitigen Relativstellung herangezogen werden. Betrachtet man beispielsweise die Außenrotation und Innenrotation eines Gelenks, so kann die Achse, um die die Rotation erfolgt ebenfalls als Symmetrieachse angesehen werden. Wird diese für beide Seiten bestimmt, so kann beispielsweise eine Außenrotation des zweiten zweitseitigen Gelenks, so beispielsweise der rechten Tibia um einen bestimmten Winkel in einem bestimmten Drehsinn durch Anwendung der Symmetrieüberlegungen in eine entsprechende Außenrotation um den selben Winkel des zweiten erstseitigen Körperteils, also beispielsweise der linken Tibia im entgegengesetzten Drehsinn umgerechnet werden. Als Symmetrieachse der Rotation kann für die jeweilige Seite beispielsweise die bereits bestimmte Tibia-Achse herangezogen werden.

Das erfindungsgemäße Navigationssystem ist schematisch ebenfalls in Figur 2 gezeigt. Die Detektionseinrichtung 300 detektiert Signale von den Markereinrichtungen 22r, 12r, 101 und 221 und führt die Detektionssignale der Datenverarbeitungseinrichtung 400 durch. Diese führt das erfindungsgemäße Verfahren durch und zeigt Anzeigesignale am Monitor 500 an.

Figur 5 kann auch als ein Beispiel für eine andere Ausführungsform der Erfindung herangezogen werden, bei der die Lagen des rechten und linken Oberarms und Unterarms gegeben sind und man feststellen möchte, ob der Übergang von voller Extension zur 90°-Beugung symmetrisch für beide Seiten erfolgt. Hierzu kann man einfach die Relativvektoren einer Seite, die eine Relativgröße darstellen, die die Relativlage beschreibt, an der medianen Sagittalebene 200 spiegeln. Das heißt die Vektoren we und wa, die sich auf der rechten Seite befinden, beschreiben die Stellungs-Relativlage des rechten Unterarms zwischen der Lage in der Stellung vor der Extension und der Lage in der Stellung bei 90°-Beugung. Die Vektoren we und wa können dann an der medianen Sagittalebene gespiegelt werden. Die gespiegelten Vektoren sind die Vektoren w'a und w'e. Sie beschreiben, wie die Stellungs-Relativlage auf der linken Seite wäre, wenn der Körper idealsymmetrisch ist. Sie beschreiben also die von rechts nach links gespiegelte Stellungsrelativlage.

Man nimmt an, dass bei dem in Figur 5 gezeigten Beispiel erstseitige Körperteildaten vorgegeben sind, die für die volle Extension die Lage 601 für den linken Unterarm beschreiben und für die 90°-Beugung die Lage 601 beschreiben. In diesem Fall ist der gespiegelte Relativvektor w'e identisch mit einem sich auf der linken Seite befindlichen Relativvektor, der das Ende des Achsabschnittes 601 mit dem Ende des Achsabschnitts 60'l verbindet. Der gespiegelte Vektor w'a ist identisch mit dem Vektor, der den Anfang des Achsabschnitts 601 mit dem Anfang des Achsabschnitts 60'l verbindet. Die in Figur 5 gezeigte Anordnung wäre demnach idealsymmetrisch bezüglich der Stellungs-Relativlage des Unterarms von voller Extension zu 90°-Beugung. In der Realität können natürlich Abweichungen auftreten, die durch Anzeige der gespiegelten Relativvektoren und der tatsächlichen Relativvektoren auf der linken Seite sichtbar gemacht werden können. Insbesondere können aus der Differenz der gespiegelten Vektoren mit den auf der linken Seite vorliegenden Relativvektoren Größen berechnet werden, die einen Wert für die vorliegende Symmetrie beim Übergang von voller Extension zu 90°-Beugung darstellen.

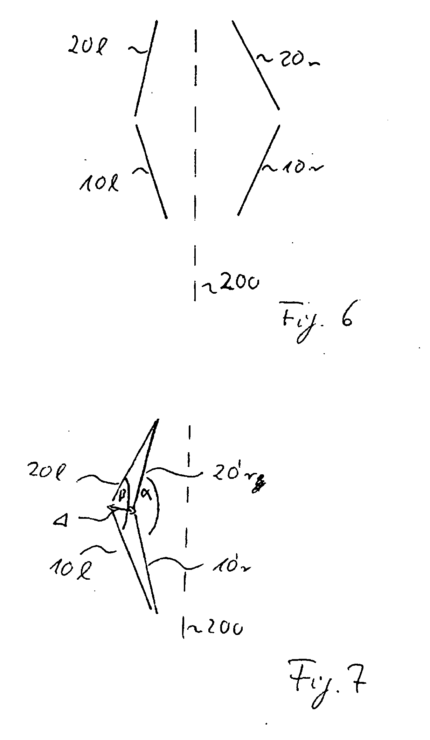

Figur 6 zeigt einen Fall, bei dem eine derartige ideale Symmetrie nicht gegeben ist. Das rechte und linke Bein soll in Extension sein. Der Patient hat einen unterschiedlich stark ausgeprägten Varus. Der Varus auf der rechten Seite ist stärker als auf der linken Seite. Um die Abweichung des rechtseitigen Varus vom linksseitigen Varus bestimmen zu können, wird erfindungsgemäß der Femur 10r und die Tibia 20r an der medianen Sagittalebene 200 gespiegelt, so dass sich die in Figur 7 gezeigte Situation ergibt. Die gespiegelte rechte Tibia ist mit 20'r bezeichnet und der gespiegelte rechte Femur mit 10'r. Der linke Femur ist mit 101 bezeichnet und die linke Tibia ist mit 201 bezeichnet. Bei einer erfindungsgemäßen Ausführungsform werden Streckenabschnitte 20'r, 10'r, 101 und 201 auf einem Monitor angezeigt, um einen Hinweis auf Abweichungen von einer idealsymmetrischen Anordnung der Körperteile zu geben. Insbesondere ist erkennbar, dass die ungespiegelten linken Körperteile in ihrer Lage von den gespiegelten rechten Körperteilen abweichen. Diese Abweichung kann auch durch Relativgrößen beschrieben werden. Beispielsweise kann ein Winkel α zwischen den gespiegelten Streckenabschnitten 20'r und 10'r bestimmt werden. Ein entsprechender Winkel kann zwischen den Streckenabschnitten 201 und 101 bestimmt werden. Die Winkeldifferenz stellt dann eine Relativgröße dar, die einen Hinweis darauf gibt, wie starke eine Abweichung von der idealsymmetrischen Anordnung der Körperteile gegeben ist. Alternativ kann auch eine Strecke Δ bestimmt werden, die beispielsweise diejenigen Endpunkte der Strecke 10r und 10'r verbindet, die jeweils der Strecke 201 und 20'r am nächsten sind. Je größer die Strecke Δ, desto größer die Abweichung von der idealsymmetrischen Anordnung.

## Patentansprüche

1. Verfahren zum Berechnen der Lage von sich anatomisch entsprechenden Körperteilen (10, 20), unter Berücksichtigung der anatomischen Symmetrie,
wobei ein erstes und zweites erstseitiges Körperteil (101, 201) an der ersten Seite eines anatomischen Körpers vorgesehen sind und dort durch ein erstseitiges Gelenk verbunden sind;
wobei ein erstes und zweites zweitseitiges Körperteil (10r, 20r) an der zweiten Seite des anatomischen Körpers vorgesehen sind und dort durch ein zweitseitiges Gelenk verbunden sind, wobei das Verfahren von einem Computer durchgeführt wird und folgende Schritte umfasst:
zweitseitige Relativlagedaten, die die relative Lage des ersten zweitseitigen Körperteils (10r) relativ zum zweiten zweitseitigen Köperteils (20r, 20'r) jeweils für eine erste (10r, 20r) und zweite (10r, 20'r) zweitseitige Relativstellung beschreiben, werden bereitgestellt;
erstseitige Relativlagedaten, die die relative Lage des ersten erstseitigen Körperteils (101) relativ zum zweiten erstseitigen Körperteils (201) in einer ersten (101, 201) erstseitigen Relativstellung beschreiben, werden bereitgestellt;
basierend auf den erstseitigen Relativlagedaten und den zweitseitigen Relativlagedaten wird die relative Lage des ersten erstseitigen Körperteils (101) zum zweiten erstseitigen Körperteil (20'1) in einer zweiten erstseitigen Relativstellung (101, 20'1), berechnet, wobei berücksichtigt wird, dass die zweite zweitseitige Relativstellung (10r, 20'r) symmetrisch zur zweiten erstseitigen Relativstellung sein soll und die erste zweitseitige Relativstellung (10r, 20r) symmetrisch zu ersten erstseitigen Relativstellung (101, 201) ist.

2. Verfahren nach Anspruch 1, bei welchem die zweite erstseitige Relativstellung unter der Annahme berechnet wird, dass die erste erstseitige Relativstellung (101, 201) symmetrisch zu der ersten zweitseitigen Relativstellung (10r, 20r) ist und/oder die erstseitigen und zweitseitigen Relativlagedaten die Lagen der ersten und zweiten erstseitigen und zweitseitigen Körperteile für die erste und zweite zweitseitige Relativstellung und für die erste erstseitige Relativstellung und die Lage des ersten erstseitigen Körperteils in der zweiten erstseitigen Relativstellung sowie die Lage einer Körpersymmetrieebene umfassen.

3. Verfahren nach Anspruch 1 oder 2, bei welchem zur Bestimmung der zweiten erstseitigen Relativstellung (101, 20'1) Symmetrieregeln angewendet werden, die die Symmetrie der Bewegung der erstseitigen Körperteile um das erstseitige Gelenk zu der Bewegung der zweitseitigen Körperteile um das zweitseitige Gelenk berücksichtigen.

4. Verfahren nach einem der Ansprüche 1 bis 3, bei welchem die Lage von Symmetrieebenen (200) und/oder Symmetrieachsen des Körpers bereitgestellt wird oder bestimmt wird, bezüglich denen die Bewegung des Gelenks symmetrisch ist, wobei basierend auf den bestimmten oder bereitgestellten Symmetrieebenen (200) und/oder Symmetrieachsen in Verbindung mit den Symmetrieregeln die zweite erstseitige Relativstellung (101, 20'1) bestimmt wird, die symmetrisch zu der zweiten zweitseitigen Relativstellung ist.

5. Verfahren nach einem der Ansprüche 1 bis 4, bei welchem Bereichsdaten bereitgestellt werden, die die relative Lage eines ersten Bereichs (10B) des ersten erstseitigen Körperteils relativ zu einem zweiten Bereich (20B, 20'B) des zweiten erstseitigen Körperteils beschreiben, wobei eine erste Relativgröße, die auf die relative Lage des ersten Bereichs zum zweiten Bereich hinweist, aus den Bereichsdaten für die erste erstseitige Relativstellung (101, 201) berechnet wird, und eine zweite Relativgröße aus den Bereichsdaten für die zweite erstseitigen Relativstellung (101, 20'1) berechnet wird.

6. Verfahren nach Anspruch 5, bei welchem die Bereichsdaten aus der Lage des ersten und/oder zweiten Bereichs in einer weiteren erstseitigen Relativstellung berechnet werden, die weder mit der ersten erstseitigen Relativstellung noch mit der zweiten erstseitigen Relativstellung übereinstimmt, wobei die Berechnung der ersten und/oder zweiten Bereichsdaten basierend auf der Lage des ersten Körperteils und/oder zweiten Körperteils in der weiteren erstseitigen Relativstellung relativ zu deren Lage in der ersten und/oder zweiten erstseitigen Relativstellung durchgeführt wird.

7. Verfahren nach einem der Ansprüche 5 oder 6, bei welchem die Bereichsdaten sich ändern und während des Vorgangs des Änderns der Vergleich erfolgt, wobei angezeigt (500) wird, wenn die ersten und zweiten Relativgrößen zumindest in etwa gleich sind.

8. Verfahren nach einem der vorhergehenden Ansprüche, bei welchem zumindest ein Teil der Daten auf der Detektion (300) der Lage der Körperteile und/oder Bereiche beruht, um so aktuelle Daten bereitzustellen, und die Berechnungen mit den aktuellen Daten durchgeführt wird.

9. Verfahren nach einem der vorhergehenden Ansprüche, bei welchem aus der Lage der zweitseitigen Körperteile (10r, 20r, 20'r) in der ersten und zweiten zweitseitigen Relativstellung, insbesondere auch in weiteren zweitseitigen Relativstellungen ein zweitseitiger Ablauf und/oder zweitseitiger Bewegungsbereich der Relativbewegung des ersten zweitseitigen Körperteils (10r) relativ zum zweiten zweitseitigen Körperteils (20r, 20'r) bestimmt und insbesondere gespeichert wird und wobei basierend auf Signaldaten, die auf Detektion (300) der Lage des ersten und zweiten erstseitigen Körperteils (101, 201, 20'1) beruhen, bestimmt wird, ob ein aus den Signaldaten bestimmter erstseitiger Ablauf und/oder erstseitiger Bewegungsbereich der Relativbewegung der erstseitigen Körperteile mit dem zweitseitigen Ablauf und/oder zweitseitigen Bewegungsbereich zumindest in einem vorbestimmten Umfang übereinstimmt und/oder ob der erstseitige Ablauf und/oder Bewegungsbereich vom zweitseitigen Ablauf und/oder Bewegungsbereich zumindest in einem bestimmten Umfang abweicht.

10. Verfahren nach einem der vorhergehenden Ansprüche, bei welchem die Lage eines Körperteils auf zumindest eine der folgenden Arten beschrieben wird:
durch die Lage einer charakteristischen Achse und/oder Ebene des Körperteils,
durch die Lage eines das Körperteil repräsentierenden generischen Modells oder geometrischen Grundkörpers,
durch die Lage von Landmarken des Körperteils.

11. Programm, das, wenn es auf einem Computer (400) läuft oder in einen Computer (400) geladen wird, den Computer (400) veranlasst, ein Verfahren zum Berechnen der Lage von sich anatomisch entsprechenden Körperteilen (10, 20) unter Berücksichtigung der anatomischen Symmetrie, auszuführen,
wobei ein erstes und zweites erstseitiges Körperteil (101, 201) an der ersten Seite eines anatomischen Körpers vorgesehen sind und dort durch ein erstseitiges Gelenk verbunden sind;
wobei ein erstes und zweites zweitseitiges Körperteil (10r, 20r) an der zweiten Seite des anatomischen Körpers vorgesehen sind und dort durch ein zweitseitiges Gelenk verbunden sind, wobei das Verfahren folgende Schritte umfasst:
zweitseitige Relativlagedaten, die die relative Lage des ersten zweitseitigen Körperteils (10r) relativ zum zweiten zweitseitigen Köperteils (20r, 20'r) jeweils für eine erste (10r, 20r) und zweite (10r, 20'r) zweitseitige Relativstellung beschreiben, werden bereitgestellt;
erstseitige Relativlagedaten, die die relative Lage des ersten erstseitigen Körperteils (101) relativ zum zweiten erstseitigen Körperteils (201) in einer ersten (101, 201) erstseitigen Relativstellung beschreiben, werden bereitgestellt;
basierend auf den erstseitigen Relativlagedaten und den zweitseitigen Relativlagedaten wird die relative Lage des ersten erstseitigen Körperteils (101) zum zweiten erstseitigen Körperteil (20'1) in einer zweiten erstseitigen Relativstellung (101, 20'1), berechnet, wobei berücksichtigt wird, dass die zweite zweitseitige Relativstellung (10r, 20'r) symmetrisch zur zweiten erstseitigen Relativstellung sein soll und die erste zweitseitige Relativstellung (10r, 20r) symmetrisch zu ersten erstseitigen Relativstellung (101, 201) ist.

12. Programm nach dem vorhergehenden Anspruch, bei dem das auszuführende Verfahren zusätzlich die Merkmale eines der Ansprüche 2 bis 10 umfasst.

13. Speichermedium, auf dem das Programm nach Anspruch 11 oder 12 gespeichert ist

14. Signalwelle, die Information überträgt, die dem Programm nach Anspruch 11 oder 12 entspricht.

15. Computer (400), auf dem das Programm nach Anspruch 11 oder 12 läuft oder gespeichert ist.

16. Navigationssystem
mit einer Detektionseinrichtung (300), die durch Detektion von Markern (221, 22r, 121, 12r) und/oder Pointer Detektionssignale erzeugt;
mit dem Computer (400) nach Anspruch 14, die die Detektionssignale verarbeitet, um daraus die Daten zu gewinnen, die zur Berechnung der Lage von Körperteilen durch das Programm erforderlich sind.

## Claims

1. Method of calculating the position of anatomically co-operating body parts (10, 20) taking account of the anatomical symmetry,
whereby a first and second first-end body part (101, 201) are disposed at the first end of an anatomical body, where they are connected by a first-end joint;
whereby a first and second second-end body part (10r, 20r) are disposed at the second end of the anatomical body, where they are connected by a second-end joint, and the method is run by a computer and comprises the following steps:
second-end relative position data describing the relative position of the first second-end body part (10r) relative to the second second-end body part (20r, 20'r) is loaded for a first (10r, 20r) and second (10r, 20'r) second-end relative position respectively;
first-end relative position data describing the relative position of the first first-end body part (101) relative to the second first-end body part (201) in a first (101, 201) first-end relative position is loaded;
the position of the first first-end body part (101) relative to the second first-end body part (20'1) in a second first-end relative position (101, 20'1) is calculated on the basis of the first-end relative position data and second-end relative position data, and allowance is made for the fact that the second second-end relative position (10r, 20'r) should be symmetrical with respect to the second first-end relative position and the first second-end relative position (10r, 20r) is symmetrical with respect to the first first-end relative position (101, 201).

2. Method as claimed in claim 1, whereby the second first-end relative position is calculated on the assumption that the first first-end relative position (101, 201) is symmetrical with respect to the first second-end relative position (10r, 20r) and/or the first-end and second-end relative position data contains the positions of the first and second first-end and second-end body parts for the first and second second-end relative position and for the first first-end relative position and the position of the first first-end body part in the second first-end relative position as well as the position of a body plane of symmetry.

3. Method as claimed in claim 1 or 2, whereby the second first-end relative position (101, 20'1) is determined by means of rules of symmetry which make allowance for the symmetry of the movement of the first-end body parts around the first-end joint with respect to the movement of the second-end body parts around the second-end joint.

4. Method as claimed in one of claims 1 to 3, whereby the position of planes of symmetry (200) and/or axes of symmetry of the body, by reference to which the movement of the joint is symmetrical, are loaded or determined, and the second first-end relative position (101, 20'1) which is symmetrical with respect to the second second-end relative position is determined on the basis of the determined or loaded planes of symmetry (200) and/or axes of symmetry in conjunction with the rules of symmetry.

5. Method as claimed in one of claims 1 to 4, whereby area data describing the relative position of a first area (10B) of the first first-end body part relative to a second area (20B, 20'B) of the second first-end body part is loaded, and a first relative size indicating the relative position of the first area with respect to the second area is calculated from the area data for the first first-end relative position (101, 201), and a second relative size is calculated from the area data for the second first-end relative position (101, 20'1).

6. Method as claimed in claim 5, whereby the area data is calculated from the position of the first and/or second area in another first-end relative position which coincides with neither the first first-end relative position nor the second first-end relative position, and the calculation of the first and/or second area data is run on the basis of the position of the first body part and/or second body part in the other first-end relative position relative to its position in the first and/or second first-end relative position.

7. Method as claimed in one of claims 5 or 6, whereby the area data varies and a comparison is run during the course of the variation, and a display (500) is generated when the first and second relative sizes are at least approximately the same.

8. Method as claimed in one of the preceding claims, whereby at least some of the data is based on detection (300) of the body parts and/or areas in order to make current data available and the calculations are run using the current data.

9. Method as claimed in one of the preceding claims, whereby a second-end sequence and/or second-end range of movement of the relative movement of the first second-end body part (10r) relative to the second second-end body part (20r, 20'r) is determined from the position of the second-end body parts (10r, 20r, 20'r) in the first and second second-end relative position, including in particular other second-end relative positions, and specifically stored, and it is then determined on the basis of signal data obtained by detecting (300) the position of the first and second first-end body part (101, 201, 20'1) whether a specific first-end sequence and/or first-end range of movement of the relative position of the first-end body parts determined from the signal data matches the second-end sequence and/or second end range of movement at least to a pre-defined degree and/or whether the first-end sequence and/or range of movement is at variance with the second-end sequence and/or range of movement at least to a specific degree.

10. Method as claimed in one of the preceding claims, whereby the position of a body part is described in at least one of the following ways:
by the position of a characteristic axis and/or plane of the body part,
by the position of a generic model or geometric body representing the body part,
by the position of landmarks of the body part.

11. Programme which, when run on a computer (400) or loaded onto a computer (400), enables the computer (400) to implement a method of calculating the position of anatomically co-operating body parts (10, 20) making allowance for the anatomical symmetry,
and a first and second first-end body part (101, 201) are disposed at the first end of an anatomical body, where they are connected by a first-end joint,
and a first and second second-end body part (10r, 20r) are disposed at the second end of the anatomical body, where they are connected by a second-end joint, and the method comprises the following steps:
second-end relative position data describing the relative position of the first second-end body part (10r) relative to the second second-end body part (20r, 20'r) are loaded for a first (10r, 20r) and second (10r, 20'r) second-end relative position respectively;
first-end relative position data describing the relative position of the first first-end body part (101) relative to the second first-end body part (201) in a first (101, 201) first-end relative position is loaded;
the relative position of the first first-end body part (101) with respect to the second first-end body part (20'1) in a second first-end relative position (101, 20'1) is calculated on the basis of the first-end relative position data and second-end relative position data, and allowance is made for the fact that the second second-end relative position (10r, 20'r) should be symmetrical with respect to the second first-end relative position and the first second-end relative position (10r, 20r) is symmetrical with respect to the first first-end relative position (101, 201).

12. Programme as claimed in the preceding claim, whereby the method to be implemented further comprises the features as claimed in one of claims 2 to 10.

13. Memory medium on which the programme as claimed in claim 11 or 12 is stored.

14. Signal wave transmitting information which co-operates with the programme as claimed in claim 11 or 12.

15. Computer (400) on which the programme as claimed in claim 11 or 12 runs or is stored.

16. Navigation system
incorporating a detection system (300) which generates detection signals by detecting markers (221, 22r, 121, 12r) and/or pointers;
incorporating the computer (400) as claimed in claim 14, which processes the detection signals in order to obtain from them the data needed for the position of body parts to be calculated by means of the programme.

## Revendications

1. Procédé pour calculer la position de parties corporelles anatomiquement correspondantes (10, 20) en tenant compte de la symétrie anatomique,
dans lequel une première et une seconde partie corporelle de premier côté (101, 201) sont prévues sur le premier côté d'un corps anatomique et y sont reliées par l'intermédiaire d'une articulation de premier côté,
dans lequel une première et une seconde partie corporelle de second côté (10r, 20r) sont prévues sur le second côté du corps anatomique et y sont reliées par l'intermédiaire d'une articulation de second côté, dans lequel le procédé est mis en oeuvre par un ordinateur et comporte les étapes suivantes consistant à :
fournir des données de position relative du second côté qui décrivent la position relative de la première partie corporelle du second côté (10r) par rapport à la seconde partie corporelle du second côté (20r, 20'r) respectivement pour un premier (10r, 20r) et un second (10r, 20'r) placement relatif du second côté,
fournir des données de position relative du premier côté qui décrivent la position relative de la première partie corporelle du premier côté (101) par rapport à la seconde partie corporelle du premier côté (201) dans un premier placement relatif du premier côté (101, 201),
calculer, sur la base des données de position relative du premier côté et des données de position relative du second côté, la position relative de la première partie corporelle du premier côté (101) par rapport à la seconde partie corporelle du premier côté (20'1) dans un second placement relatif du premier côté (101, 20'1), en tenant compte du fait que le second placement relatif du second côté (10r, 20'r) doit être symétrique au second placement relatif du premier côté et que le premier placement relatif du second côté (10r, 20r) est symétrique au premier placement relatif du premier côté (101, 201).

2. Procédé selon la revendication 1, dans lequel on calcule le second placement relatif du premier côté en supposant que le premier placement relatif du premier côté (101, 201) est symétrique au premier placement relatif du second côté (10r, 20r) et/ou les données de position relative du premier côté et du second côté incluent les positions des première et seconde parties corporelles du premier côté et du second côté pour les premier et second placements relatifs du second côté et pour le premier placement relatif du premier côté et la position de la première partie corporelle du premier côté dans le second placement relatif du premier côté ainsi que la position d'un plan de symétrie du corps.

3. Procédé selon la revendication 1 ou 2, dans lequel on utilise des règles de symétrie pour déterminer le second placement relatif du premier côté (101, 20'1), lesquelles règles tiennent compte de la symétrie du mouvement des parties corporelles du premier côté autour de l'articulation du premier côté par rapport au mouvement des parties corporelles du second côté autour de l'articulation du second côté.

4. Procédé selon l'une quelconque des revendications 1 à 3, dans lequel on fournit ou on détermine la position de plans de symétrie (200) et/ou d'axes de symétrie du corps, par rapport auxquels le mouvement de l'articulation est symétrique, dans lequel, sur la base des plans de symétrie (200) et/ou des axes de symétrie déterminés ou fournis, on détermine le second placement relatif du premier côté (101, 20'1) conjointement avec les règles de symétrie, lequel second placement relatif du premier côté est symétrique au second placement relatif du second côté.

5. Procédé selon l'une quelconque des revendications 1 à 4, dans lequel on fournit des données de régions qui décrivent la position relative d'une première région (10B) de la première partie corporelle du premier côté par rapport à une seconde région (20B, 20'B) de la seconde partie corporelle du premier côté, dans lequel on calcule une première grandeur relative qui indique la position relative de la première région par rapport à la seconde région, à partir des données de régions pour le premier placement relatif du premier côté (101, 201), et on calcule une seconde grandeur relative à partir des données de régions pour le second placement relatif du premier côté (101, 20'1).

6. Procédé selon la revendication 5, dans lequel on calcule les données de régions à partir de la position de la première et/ou seconde région dans un autre placement relatif du premier côté qui correspond au premier placement relatif du premier côté ou au second placement relatif du premier côté, dans lequel on effectue le calcul des premières et/ou secondes données de régions sur la base de la position de la première partie corporelle et/ou de la seconde partie corporelle dans l'autre placement relatif du premier côté par rapport à leur position dans le premier et/ou second placement relatif du premier côté.

7. Procédé selon l'une quelconque des revendications 5 ou 6, dans lequel les données de régions varient et la comparaison est effectuée pendant le processus de variation, dans lequel on affiche (500) lorsque les première et seconde grandeurs relatives sont au moins approximativement identiques.

8. Procédé selon l'une quelconque des revendications précédentes, dans lequel au moins une partie des données est basée sur la détection (300) de la position des parties corporelles et/ou des régions de manière à fournir des données actuelles, et les calculs sont effectués en utilisant les données actuelles.

9. Procédé selon l'une quelconque des revendications précédentes, dans lequel, à partir de la position des parties corporelles du second côté (10r, 20r, 20'r) dans les premier et second placements relatifs du second côté, en particulier également dans d'autres placements relatifs du second côté, on détermine et on mémorise en particulier une trajectoire du second côté et/ou une plage de mouvement du mouvement relatif du second côté de la première partie corporelle du second côté (10r) par rapport à la seconde partie corporelle du second côté (20r, 20'r), et dans lequel on détermine, sur la base de signaux de données qui sont basés sur la détection (300) de la position de la première et de la seconde partie corporelle du premier côté (101, 201, 20'1), si une trajectoire du premier côté et/ou une plage de mouvement du mouvement relatif du premier côté de la partie corporelle du premier côté, déterminée à partir des signaux de données, correspond à la trajectoire du second côté et/ou à la plage de mouvement du second côté au moins sur une étendue prédéterminée, et/ou si la trajectoire du premier côté et/ou la plage de mouvement s'écarte de la trajectoire et/ou de la plage de mouvement du second côté au moins sur une étendue prédéterminée.

10. Procédé selon l'une quelconque des revendications précédentes, dans lequel on décrit la position d'une partie corporelle selon au moins une des manières suivantes :
par la position d'un axe caractéristique et/ou d'un plan de la partie corporelle,
par la position d'un modèle générique représentant la partie corporelle ou d'un corps de base géométrique,
par la position de points de repère sur la partie corporelle.

11. Programme qui, lorsqu'il tourne sur un ordinateur (400) ou lorsqu'il est chargé sur un ordinateur (400), amène l'ordinateur (400) à mettre en oeuvre un procédé pour calculer la position de parties corporelles anatomiquement correspondantes (10, 20) en tenant compte de la symétrie anatomique,
dans lequel une première et une seconde partie corporelle de premier côté (101, 201) sont prévues sur le premier côté d'un corps anatomique et y sont reliées par l'intermédiaire d'une articulation de premier côté,
dans lequel une première et une seconde partie corporelle de second côté (10r, 20r) sont prévues sur le second côté du corps anatomique et y sont reliées par l'intermédiaire d'une articulation de second côté, dans lequel le procédé comporte les étapes suivantes consistant à :
fournir des données de position relative du second côté qui décrivent la position relative de la première partie corporelle du second côté (10r) par rapport à la seconde partie corporelle du second côté (20r, 20'r) respectivement pour un premier (10r, 20r) et un second (10r, 20'r) placement relatif du second côté,
fournir des données de position relative du premier côté qui décrivent la position relative de la première partie corporelle du premier côté (101) par rapport à la seconde partie corporelle du premier côté (201) dans un premier placement relatif du premier côté (101, 201),
calculer, sur la base des données de position relative du premier côté et des données de position relative du second côté, la position relative de la première partie corporelle du premier côté (101) par rapport à la seconde partie corporelle du premier côté (20'1) dans un second placement relatif du premier côté (101, 20'1), en tenant compte du fait que le second placement relatif du second côté (10r, 20'r) doit être symétrique au second placement relatif du premier côté et que le premier placement relatif du second côté (10r, 20r) est symétrique au premier placement relatif du premier côté (101, 201).

12. Programme selon la revendication précédente, dans lequel le procédé à mettre en oeuvre inclut de plus les caractéristiques de l'une quelconque des revendications 2 à 10.

13. Support de mémorisation sur lequel le programme selon la revendication 11 ou 12 est mémorisé.

14. Onde de signal transportant des informations correspondant au programme selon la revendication 11 ou 12.

15. Ordinateur (400) sur lequel le programme selon la revendication 11 ou 12 tourne ou est mémorisé.

16. Système de navigation comportant :
un dispositif de détection (300) qui génère des signaux de détection par la détection de marqueurs (221, 22r, 121, 12r) et/ou d'un pointeur,
l'ordinateur (400) selon la revendication 14 qui traite les signaux de détection de manière à acquérir les données requises pour calculer la position de parties corporelles par l'intermédiaire du programme.
